(19) Europäisches Patentamt
European Patent Office
Office européen des brevets

(11) Veröffentlichungsnummer: **0 253 084 A2**

(12) **EUROPÄISCHE PATENTANMELDUNG**

(21) Anmeldenummer: **87106381.4**

(22) Anmeldetag: **30.04.87**

(51) Int. Cl.⁴: **C 07 D 233/64**, C 07 D 233/84, C 07 D 233/88, C 07 D 233/56, C 07 D 413/04, C 07 D 235/06, C 07 D 235/24, C 07 D 233/70, A 61 K 31/415, A 61 K 31/535

(30) Priorität: **13.05.86 CH 1940/86**

(43) Veröffentlichungstag der Anmeldung: **20.01.88 Patentblatt 88/3**

(84) Benannte Vertragsstaaten: **AT BE CH DE FR GB IT LI NL**

(71) Anmelder: **F. HOFFMANN-LA ROCHE & CO. Aktiengesellschaft, CH-4002 Basel (CH)**

(72) Erfinder: **Klötzer, Wilhelm, Prof. Dr., Löfflerweg 5, A-6020 Innsbruck (AT)**
Erfinder: **Link, Helmut, Dr., Dammerkirchstrasse 70, CH-4056 Basel (CH)**
Erfinder: **Müssner, Renate, Schneeburggasse 67a, A-6020 Innsbruck (AT)**
Erfinder: **Schiestl, Werner, Dr., Ampfererstrasse 42, A-6020 Innsbruck (AT)**
Erfinder: **Singewald, Nicolas, Unterbergerstrasse 14, A-6020 Innsbruck (AT)**

(74) Vertreter: **Notegen, Eric-André et al, Grenzacherstrasse 124 Postfach 3255, CH-4002 Basel (CH)**

(54) **1,3-Disubstituierte Imidazoliumsalze.**

(57) Die neuen Imidazoliumverbindungen der allgemeinen Formel

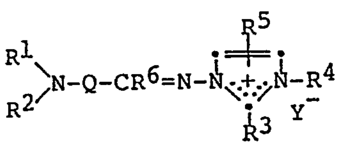

I

worin das Symbol Q Arylen oder Heteroarylen, die Gruppe $-NR^1R^2$ eine basische Aminogruppe, $R^3$ niederes Alkylthio, niederes Alkoxy oder die Gruppe $-(A)_n-Ra$, $R^4$ eine gesättigte oder partiell ungesättigte niedere Kohlenwasserstoffgruppe, eine basische Aminogruppe oder die Gruppe -N=CRc-Rb, -CHRcRd, -NH-CHRcRd, -NH-CO-Re oder -CHRc-CO-Re, $R^5$ Wasserstoff, niederes Alkyl, niederes Hydroxyalkyl, niederes Alkoxyalkyl, niederes Halogenalkyl, Aryl oder einen ankondensierten Benzolring, $R^6$ Wasserstoff oder niederes Alkyl, Ra und Rb je Aryl, Heteroaryl oder eine basische Aminogruppe, Rc Wasserstoff oder niederes Alkyl, Rd Aryl oder Heteroaryl, Re Wasserstoff, eine gegebenenfalls, über ein Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl- oder basische Aminogruppe, A Vinylen oder niederes Alkylen, n die Zahl 0 oder 1, die gestrichelte Linie eine zusätzliche Doppelbindung und das Symbol $Y^-$ ein pharmazeutisch annehmbares Anion bedeuten, und die pharmazeutisch annehmbaren Säureadditionssalze davon, besitzen wertvolle pharmakologische Eigenschaften. Sie besitzen insbesondere antibakterielle, antimykotische, protozoozide und/oder anthelmintische Eigenschaften und sind insbesondere wirksam gegen parasitäre Protozoen und Würmer. Sie können nach an sich bekannten Methoden hergestellt werden.

EP 0 253 084 A2

0253084

F. HOFFMANN-LA ROCHE & CO. Aktiengesellschaft, Basel/Schweiz

RAN 4472/6

# 1,3-Disubstituierte Imidazoliumsalze

Die vorliegende Erfindung betrifft neue Imidazoliumverbindungen der allgemeinen Formel

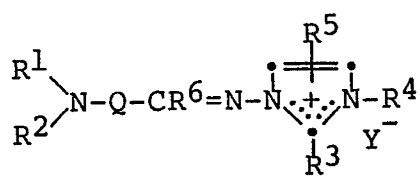

I

worin das Symbol Q Arylen oder Heteroarylen, die Gruppe $-NR^1R^2$ eine basische Aminogruppe, $R^3$ niederes Alkylthio, niederes Alkoxy oder die Gruppe $-(A)_n-Ra$, $R^4$ eine gesättigte oder partiell ungesättigte niedere Kohlenwasserstoffgruppe, eine basische Aminogruppe oder die Gruppe -N=CRc-Rb, -CHRcRd, -NH-CHRcRd, -NH-CO-Re oder -CHRc-CO-Re, $R^5$ Wasserstoff, niederes Alkyl, niederes Hydroxyalkyl, niederes Alkoxyalkyl, niederes Halogenalkyl, Aryl oder einen ankondensierten Benzolring, $R^6$ Wasserstoff oder niederes Alkyl, Ra und Rb je Aryl, Heteroaryl oder eine basische Aminogruppe, Rc Wasserstoff oder niederes Alkyl, Rd Aryl oder Heteroaryl, Re Wasserstoff, eine gegebenenfalls, über ein

Nt/19.2.87

Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl- oder basische Aminogruppe, A Vinylen oder niederes Alkylen, n die Zahl 0 oder 1, die gestrichelte Linie eine zusätzliche Doppelbindung und das Symbol $Y^-$ ein pharmazeutisch annehmbares Anion bedeuten,

und die pharmazeutisch annehmbaren Säureadditionssalze davon. Diese neuen Verbindungen besitzen wertvolle pharmakologische Eigenschaften. Sie besitzen insbesondere antibakterielle, antimykotische, protozoozide und/oder anthelmintische Eigenschaften.

Gegenstand der vorliegenden Erfindung sind: Die obigen Verbindungen der Formel I und die pharmazeutisch annehmbaren Säureadditionssalze davon als solche; ein Verfahren und Zwischenprodukte zu deren Herstellung; die Verwendung der Zwischenprodukte zur Herstellung von therapeutischen Wirkstoffen; die obigen Verbindungen der Formel I und die pharmazeutisch annehmbaren Säureadditionssalze davon zur Anwendung als therapeutische Wirkstoffe; Arzneimittel auf der Basis von Verbindungen der Formel I und deren pharmazeutisch annehmbaren Säureadditionssalzen und deren Herstellung; die Verwendung dieser Verbindungen bei der Bekämpfung oder Verhütung von Krankheiten; sowie die Verwendung dieser Verbindungen zur Herstellung von antibakteriell, antimykotisch, protozoozid und/oder anthelmintisch wirksamen Mitteln.

Der Ausdruck "nieder" bezeichnet Reste und Verbindungen mit höchstens 7, vorzugsweise höchstens 4 Kohlenstoffatomen. Der Ausdruck "Alkyl" für sich allein genommen oder in Zusammensetzungen, wie "Alkylgruppen", "Alkoxy" und "Alkylthio", bezeichnet geradkettige oder verzweigte, gesättigte Kohlenwasserstoffreste, wie Methyl, Aethyl, n-Propyl, i-Propyl, n-Butyl, s-Butyl, i-Butyl und t-Butyl.

Der Ausdruck "gesättigte oder partiell ungesättigte Kohlenwasserstoffgruppe" bezeichnet offenkettige und cyclische Gruppen und Kombinationen davon. Beispiele für gesättigte und partiell ungesättigte niedere Kohlenwasserstoffgruppen sind: Niedere Alkylgruppen, wie Methyl, Aethyl, Propyl, i-Propyl, s-Butyl und i-Butyl; niedere Alkenylgruppen, wie 2-Propenyl, 2-Butenyl, 3-Butenyl und 2-Methyl-2--propenyl; gegebenenfalls durch niedere Alkylgruppen substituierte niedere Cycloalkylgruppen, wie Cyclopropyl, Cyclopentyl, 2-Methylcyclopentyl, Cyclohexyl und 3-Methyl-cyclohexyl; gegebenenfalls durch niedere Alkylgruppen substituierte niedere Cycloalkenylgruppen, wie 3-Cyclopentenyl, 1-Methyl-3-cyclopentenyl und 3-Cyclohexenyl; durch niedere Cycloalkyl- oder Cycloalkenylgruppen substituierte niedere Alkyl- oder Alkenylgruppen, wie Cyclopropylmethyl, Cyclopropyläthyl, Cyclopentylmethyl, Cyclohexylmethyl, 2-Cyclohexenylmethyl und 3-Cyclopropyl-2-propenyl. Die bevorzugten niederen Kohlenwasserstoffgruppen sind gesättigt. Die niederen Alkyl- und niederen Cycloalkylgruppen, insbesondere die niederen Alkylgruppen, sind besonders bevorzugte niedere Kohlenwasserstoffgruppen.

Der Ausdruck "Aryl" bezeichnet carbocyclische aromatische Gruppen, vorzugsweise mono- oder bicyclische Gruppen, d.h. Phenyl- und Naphthylgruppen, insbesondere Phenylgruppen. Diese Gruppen sind gegebenenfalls durch einen, zwei oder drei Substituenten aus der Gruppe bestehend aus basischem Amino, niederem Alkyl, niederem Alkoxy, niederem Alkylthio, niederem Alkanoyl, niederem Alkoxycarbonyl, Aryl (insbesondere Phenyl), Halogen, Trifluormethyl, Hydroxy, Nitro und Cyano substituiert. Diese Gruppen sind vorzugsweise unsubstituiert oder durch einen, zwei oder drei Substituenten aus der Gruppe bestehend aus niederem Alkyl, niederem Alkoxy, niederem Alkylthio, Halogen, Nitro und Di(niederem Alkyl)Amino substituiert.

Der Ausdruck "Arylen" bezeichnet carbocyclische aromatische Gruppen mit zwei freien Valenzen, vorzugsweise mono- oder bicyclische Gruppen, d.h. Phenylen- und Naphthylengruppen, insbesondere 1,4- oder 1,2-Phenylengruppen und im speziellen 1,4-Phenylengruppen. Diese Gruppen können durch einen oder zwei Substituenten aus der Gruppe bestehend aus niederem Alkyl, niederem Alkoxy, niederem Alkylthio, niederem Alkanoyl, niederem Alkoxycarbonyl, Aryl (insbesondere Phenyl), Halogen, Trifluormethyl, Hydroxy, Nitro und Cyano substituiert sein. Sie sind vorzugsweise unsubstituiert.

Der Ausdruck "Heteroaryl" bezeichnet heterocyclische aromatische Gruppen, vorzugsweise mono- oder bicyclische Gruppen, insbesondere gegebenenfalls mit einem Benzolring annellierte 5- oder 6-gliedrige aromatische Heterocyclen, welche gegebenenfalls durch einen, zwei oder drei Substituenten aus der Gruppe bestehend aus basischem Amino, niederem Alkyl, niederem Alkoxy, niederem Alkylthio, niederem Alkanoyl, niederem Alkoxycarbonyl, Aryl (insbesondere Phenyl), Halogen, Trifluormethyl, Hydroxy, Nitro und Cyano substituiert sein können. Die 5-gliedrigen aromatischen Heterocyclen enthalten als Heteroringglieder vorzugsweise ein Sauerstoff- oder Schwefelatom oder eine Iminogruppe und gegebenenfalls noch ein oder zwei Stickstoffatome. Die 6-gliedrigen aromatischen Heterocyclen enthalten als Ringglieder vorzugsweise ein, zwei oder drei Stickstoffatome.

Der Ausdruck "Heteroarylen" bezeichnet heterocyclische aromatische Gruppen mit zwei freien Valenzen, vorzugsweise mono- oder bicyclische Gruppen, insbesondere gegebenenfalls mit einem Benzolring annellierte 5- und 6-gliedrige aromatische Heterocyclen mit zwei freien Valenzen, welche noch durch einen oder zwei Substituenten aus der Gruppe bestehend aus niederem Alkyl, niederem Alkoxy, niederem Alkylthio, niederem Alkanoyl, niederem Alkoxycarbonyl, Aryl (insbesondere Phenyl), Halogen, Trifluormethyl, Hydroxy, Nitro und

Cyano substituiert sein können.

Der Ausdruck "Halogen" bezeichnet die vier Formen Fluor, Chlor, Brom und Jod.

Der Ausdruck "basische Aminogruppe" oder "basisches Amino" bezeichnet unsubstituierte oder mono- oder disubstituierte Aminogruppen mit basischem Charakter. Die basischen Aminogruppen können durch die allgemeine Formel -NRR' dargestellt werden. Darin bedeuten vorzugsweise R Wasserstoff oder niederes Alkyl und R' Wasserstoff oder eine gesättigte oder partiell ungesättigte niedere Kohlenwasserstoffgruppe, welche gegebenenfalls durch eine oder zwei niedere Alkoxy- oder Hydroxygruppen oder durch eine Amino-, niedere Alkylamino-, niedere Dialkylamino-, Oxo-, niedere Alkoxycarbonyl- oder niedere Alkylendioxygruppe substituiert ist, oder R und R' zusammen mit dem Stickstoffatom einen gegebenenfalls durch eine oder zwei niedere Alkylgruppen substituierten 4- bis 7-gliedrigen gesättigten N-Heterocyclus, der als Ringglied anstelle einer Methylengruppe ein Sauerstoff- oder Schwefelatom oder die Gruppe >SO, $>SO_2$, >CO, >CH-Rf oder >N-Rg enthalten kann, wobei Rf Hydroxy, niederes Alkanoyl, niederes Alkoxycarbonyl, Carbamoyl, mono- oder di(niederes Alkyl)carbamoyl oder eine gegebenenfalls über ein Sauerstoffatom gebundene, gesättigte oder partiell ungesättigte niedere Kohlenwasserstoffgruppe, welche gegebenenfalls noch durch eine oder zwei niedere Alkoxy- oder Hydroxygruppen oder durch eine Amino-, niedere Alkylamino-, niedere Dialkylamino-, Oxo-, niedere Alkoxycarbonyl- oder niedere Alkylendioxygruppe substituiert ist, und Rg Wasserstoff, niederes Alkanoyl, niederes Alkoxycarbonyl, Carbamoyl, mono- oder di(niederes Alkyl)carbamoyl oder eine gesättigte oder partiell ungesättigte niedere Kohlenwasserstoffgruppe, welche gegebenenfalls durch eine oder zwei niedere Alkoxy- oder Hydroxygruppen oder durch eine Amino-, niedere Alkylamino-, niedere Dialkylamino-, Oxo-, niedere Alkoxycarbonyl- oder niedere Alkylendioxygruppe substituiert

ist, bedeuten.

Besonders bevorzugte basische Aminogruppen -NRR' sind diejenigen, worin R Wasserstoff oder niederes Alkyl und R' eine gesättigte niedere Kohlenwasserstoffgruppe oder R und R' zusammen mit dem Stickstoffatom einen gegebenenfalls durch eine Hydroxy-, niedere Alkoxy-, niedere Hydroxyalkyl- oder niedere Alkoxyalkylgruppe substituierten 4-, 5- oder 6-gliedrigen gesättigten N-Heterocyclus oder einen gegebenenfalls durch eine oder zwei niedere Alkylgruppen substituierten 6-gliedrigen gesättigten N-Heterocyclus, der anstelle einer Methylengruppe ein Sauerstoff- oder Schwefelatom oder eine Imino- oder niedere Alkyliminogruppe enthält, bedeuten.

Ganz besonders bevorzugte basische Aminogruppen -NRR' sind diejenigen, worin R und R' je niederes Alkyl oder zusammen mit dem Stickstoffatom 1-Pyrrolidinyl, 1-Piperidinyl, 4-Morpholinyl, 2,6-Dimethyl-4-morpholinyl, 4-Thiomorpholinyl oder 4-Methyl-1-piperazinyl bedeuten.

Enthält eine Verbindung der Formel I mehr als eine basische Aminogruppe, so können diese gleich oder verschieden sein.

Der Ausdruck "Abgangsgruppe" bezeichnet vorzugsweise Halogenatome, wie Chlor, Brom und Jod, niedere Alkylsulfonyloxygruppen, wie Methylsulfonyloxy, und Arylsulfonyloxygruppen, wie p-Tolylsulfonyloxy.

In einer speziellen Ausführungsform betrifft die vorliegende Erfindung Verbindungen der obigen Formel I, worin $R^3$ niederes Alkylthio bedeutet.

In einer weiteren speziellen Ausführungsform betrifft die vorliegende Erfindung Verbindungen der obigen Formel I, worin $R^3$ die Gruppen $-(A)_n-Ra$ bedeutet. Vorzugsweise

bedeutet n die Zahl 0 oder die Zahl 1, wobei A vorzugsweise Vinylen bedeutet. In einer bevorzugten Ausführungsform bedeutet Ra eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe, wobei Ra besonders bevorzugt eine unsubstituierte oder durch niederes Alkyl, niederes Alkoxy oder Halogen mono-substituierte Phenylgruppen, wie Phenyl, p-Chlorphenyl, p-Tolyl und m-Methoxyphenyl, bedeutet. In einer weiteren bevorzugten Ausführungsform bedeutet Ra die Gruppe -NRR', wobei R und R' je Wasserstoff oder niederes Alkyl oder zusammen mit dem Stickstoffatom einen gegebenenfalls durch eine oder zwei niedere Alkylgruppen substituierten, 5- oder 6-gliedrigen gesättigten Heterocyclus, der ein oder zwei Stickstoffatome oder ein Stickstoffatom und ein Sauerstoff- oder Schwefelatom als Heteroatome enthält, bedeuten. R und R' bedeuten vorzugsweise je niederes Alkyl, insbesondere Methyl, oder zusammen mit dem Stickstoffatom 4-Morpholinyl oder 1-Piperidinyl.

Das Symbol $R^5$ bedeutet vorzugsweise Wasserstoff.

In einer bevorzugten Ausführungsform bedeutet $R^4$ die Gruppe $-N{=}CR^6-Q-NR^1R^2$.

In einer weiteren bevorzugten Ausführungsform bedeutet $R^4$ die Gruppe -CHRc-CO-Re, wobei Rc vorzugsweise Wasserstoff und Re vorzugsweise niederes Alkyl, niederes Alkoxy oder eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe bedeuten. Ganz besonders bevorzugt bedeutet Re niederes Alkyl, insbesondere t-Butyl.

Das Symbol Q bedeutet vorzugsweise gegebenenfalls durch einen oder zwei Substituenten aus der Gruppe bestehend aus niederem Alkyl und niederem Alkoxy substituiertes 1,4-Phenylen. In einer besonders bevorzugten Ausführungsform bedeutet das Symbol Q 1,4-Phenylen.

$R^1$ und $R^2$ bedeuten vorzugsweise je niederes Alkyl. In einer besonders bevorzugten Ausführungsform bedeuten $R^1$ und $R^2$ je Methyl.

Das Symbol $R^6$ bedeutet vorzugsweise Wasserstoff.

Die nachfolgend aufgeführten Imidazoliumsalze sind bevorzugte Vertreter der durch die allgemeine Formel I definierten Stoffklasse:

1,3-Bis[[p-(Dimethylamino)benzyliden]amino]-2-[2-(dimethylamino)vinyl]imidazoliumchlorid,

1,3-Bis[[p-(Dimethylamino)benzyliden]amino]-2-(2-piperidinovinyl)imidazoliumchlorid,

1,3-Bis[[p-(Dimethylamino)benzyliden]amino]-2-(2-morpholinovinyl)imidazoliumchlorid,

1,3-Bis[[p-(Dimethylamino)benzyliden]amino]-2-phenylimidazoliumchlorid,

1,3-Bis[[p-(Dimethylamino)benzyliden]amino]-2-(p-chlorphenyl)imidazoliumchlorid,

3-[[p-(Dimethylamino)benzyliden]amino]-1-(pivaloylmethyl)-2-(p-tolyl)imidazoliumbromid und

3-[[p-(Dimethylamino)benzyliden]amino]-1-(pivaloylmethyl)-2-(m-methoxyphenyl)imidazoliumbromid.

Die neuen Verbindungen der Formel I und ihre pharmazeutisch annehmbaren Säureadditionssalze können erfindungsgemäss hergestellt werden, indem man

a) eine Verbindung der allgemeinen Formel

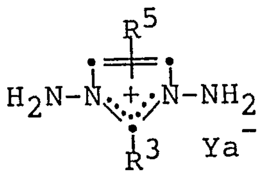 II

oder

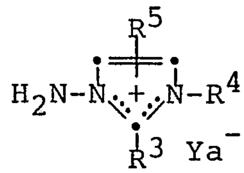 III

worin $R^3$, $R^4$, $R^5$ und die gestrichelte Linie obige Bedeutung besitzen, und $Ya^-$ ein Anion bedeutet,

mit einer Carbonylverbindung der allgemeinen Formel

$$R^1R^2N\text{-}Q\text{-}CO\text{-}R^6 \qquad IV$$

worin $R^1$, $R^2$, $R^6$ und Q obige Bedeutung besitzen,

umsetzt, oder

b) eine Verbindung der allgemeinen Formel

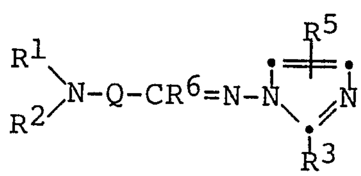 V

worin $R^1$, $R^2$, $R^3$, $R^5$, $R^6$ und Q obige Bedeutung besitzen,

mit einer Verbindung der allgemeinen Formel

$$R^{41}\text{-}X \qquad VI$$

worin $R^{41}$ eine gesättigte oder partiell ungesättigte niedere Kohlenwasserstoffgruppe oder die Gruppe -CHRcRd oder -CHRc-CO-Re und X eine Abgangsgruppe bedeuten, und Rc, Rd und Re obige Bedeutung besitzen,

umsetzt, oder

c) eine Verbindung der allgemeinen Formel

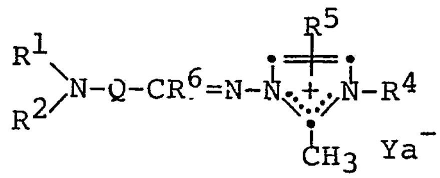

VII

worin $R^1$, $R^2$, $R^4$, $R^5$, $R^6$, Q, $Ya^-$ und die gestrichelte Linie obige Bedeutung besitzen, mit einem Aldehyd der allgemeinen Formel

Ra-CHO VIII

worin Ra obige Bedeutung besitzt, kondensiert, oder

d) eine Verbindung der allgemeinen Formel

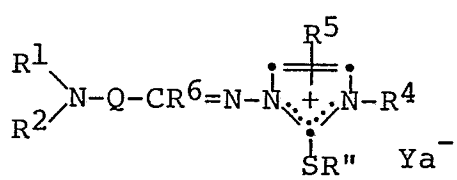

Ib

worin $R^1$, $R^2$, $R^4$, $R^5$, $R^6$, Q, $Ya^-$ und die gestrichelte Linie obige Bedeutung besitzen, und R" niederes Alkyl bedeutet, mit Ammoniak oder einem primären oder sekundären basischen Amin umsetzt,

e) gegebenenfalls in einer erhaltenen Verbindung das mit $Ya^-$ bezeichnete Anion gegen ein pharmazeutisch annehmbares Anion austauscht und

f) erwünschtenfalls eine erhaltene Verbindung der Formel I in ein pharmazeutisch annehmbares Salz überführt.

Bei verschiedenen der obigen erfindungsgemässen Verfahren ist es notwendig, die in den Ausgangsstoffen allfällig vorhandenen reaktionsfähigen Amino- und/oder Hydroxylgruppen durch Schutzgruppen zu blockieren. Diese Fälle sind für den Fachmann ohne weiteres erkennbar, und auch die Auswahl der jeweils geeigneten Schutzgruppen bereitet ihm keine Schwierigkeiten.

Bei der Umsetzung von Aminen mit Aldehyden oder Ketonen gemäss Verfahrensvariante a) handelt es sich um eine an sich bekannte und jedem Fachmann geläufige Reaktion. Als Lösungsmittel eignen sich beispielsweise Wasser, niedere Fettsäuren, wie Essigsäure und Propionsäure, niedere Alkohole, wie Methanol, Aethanol, 1-Propanol und 2-Propanol, niedere Fettsäureester, wie Essigester, niedere Aether, wie Diäthyläther, t-Butylmethyläther, Aethylenglykoldimethyläther, Tetrahydrofuran und Dioxan, halogenierte niedere Kohlenwasserstoffe, wie Methylenchlorid, Chloroform und Aethylenchlorid, aromatische Kohlenwasserstoffe, wie Benzol, Toluol und Xylol, Acetonitril, N,N-Dimethylformamid und Dimethylsulfoxid. Die Reaktionstemperatur ist nicht kritisch. Die Reaktion kann beispielsweise in einem Bereich von etwa 0°C bis zur Siedetemperatur des gewählten Lösungsmittels durchgeführt werden. Man arbeitet jedoch vorzugsweise bei Raumtemperatur. Bei der Umsetzung mit den weniger reaktiven Ketonen der Formel IV ($R^6$ = niederes Alkyl) verwendet man vorzugsweise ein Kondensationsmittel, wie Triäthyloxoniumtetrafluoroborat.

Gemäss Verfahrensvariante b) können Verbindungen der Formel I, worin $R^4$ eine gesättigte oder partiell ungesättigte niedere Kohlenwasserstoffgruppe oder die Gruppe -CHRcRd oder -CHRc-CO-Re bedeutet, durch Alkylierung von Imidazolderivaten der Formel V hergestellt werden. Es

handelt sich auch hierbei um eine an sich bekannte und jedem Fachmann geläufige Reaktion. Geeignete Lösungsmittel sind beispielsweise halogenierte niedere Kohlenwasserstoffe, wie Methylenchlorid, Chloroform und Aethylenchlorid, offenkettige oder cyclische Aether, wie Diäthyläther, t-Butylmethyläther, Aethylenglykoldimethyläther, Tetrahydrofuran und Dioxan, niedere Fettsäureester, wie Essigester, niedere Alkohole, wie Methanol, Aethanol, 1-Propanol und 2-Propanol, aromatische Kohlenwasserstoffe, wie Benzol, Toluol und Xylol, Acetonitril, N,N-Dimethylformamid und Dimethylsulfoxid. Man verwendet vorzugsweise einen niederen Alkohol, wie 2-Propanol, oder Acetonitril. Die Reaktionstemperatur ist nicht kritisch. Man kann beispielsweise in einem Bereich von etwa 0°C bis zur Siedetemperatur des Lösungsmittel, vorzugsweise in einem Bereich von etwa Raumtemperatur bis etwa 60°C, arbeiten.

Gemäss Verfahrensvariante c) können Verbindungen der Formel I, worin $R^3$ die Gruppe -CH=CH-Ra bedeutet, durch Kondensation einer Verbindung der Formel VII mit einem Aldehyd der Formel VIII hergestellt werden. Auch in diesem Fall handelt es sich um eine an sich bekannte und jedem Fachmann geläufige Reaktion. Sofern Ra Aryl oder Heteroaryl bedeutet, wird die Reaktion vorzugsweise in Gegenwart eines sekundären Amins, insbesondere eines cyclischen Amins, wie Piperidin oder Morpholin, durchgeführt. Als Lösungsmittel eignen sich beispielsweise niedere Alkohole, wie Methanol, Aethanol, 1-Propanol und 2-Propanol, aromatische Kohlenwasserstoffe, wie Benzol und Toluol, offenkettige und cyclische Aether, wie Aethylenglykoldimethyläther, Tetrahydrofuran und Dioxan, halogenierte niedere Kohlenwasserstoffe, wie Methylenchlorid, N,N-Dimethylformamid und Dimethylsulfoxid. Man arbeitet vorzugsweise bei der Siedetemperatur des gewählten Lösungsmittels. Im einer bevorzugten Ausführungsform verwendet man als Lösungsmittel einen Schlepper, beispielsweise einen aromatischen Kohlenwasserstoff oder einen halogenierten niederen Kohlenwasserstoff, und entfernt

das gebildete Reaktionswasser mit einem Wasserabscheider.

Sofern Ra eine basische Aminogruppe bedeutet, wird der Aldehyd der Formel VIII vorzugsweise in Form eines entsprechenden Di(niederen Alkyl)acetals eingesetzt, wobei die vorerwähnten Lösungsmittel auch für den vorliegenden Fall geeignet sind. Vorzugsweise verwendet man einen niederen Alkohol, wie 1-Propanol, einen halogenierten niederen Kohlenwasserstoff, wie Methylenchlorid, oder Dimethylformamid als Lösungsmittel. Die Reaktionstemperatur ist nicht kritisch. Man kann in einem Bereich von etwa Raumtemperatur bis zur Siedetemperatur des gewählten Lösungsmittels arbeiten. Vorzugsweise arbeitet man jedoch in einem Bereich von etwa Raumtemperatur bis etwa 100°C.

Gemäss Verfahrensvariante d) können Verbindungen der Formel I, worin $R^3$ eine basische Aminogruppe bedeutet, durch Umsetzen einer Verbindung der Formel Ib mit Ammoniak oder einem primären oder sekundären basischen Amin hergestellt werden. Auch in diesem Fall handelt es sich um eine an sich bekannte und jedem Fachmann geläufige Reaktion. Geeignete Lösungsmittel sind beispielsweise halogenierte niedere Kohlenwasserstoffe, wie Methylenchlorid, offenkettige und cyclische Aether, wie Diäthyläther, t-Butylmethyläther, Tetrahydrofuran und Dioxan, N,N-Dimethylformamid, Acetonitril und Dimethylsulfoxid. Bevorzugte Lösungsmittel sind N,N-Dimethylformamid und Acetonitril. Die Reaktionstemperatur ist nicht kritisch, und man kann beispielsweise in einem Bereich von etwa 0°C bis zur Siedetemperatur des gewählten Lösungsmittels arbeiten. Vorzugsweise arbeitet man jedoch bei Raumtemperatur.

Gemäss Verfahrensvariante e) kann man in einer erhaltenen Verbindung das mit $Ya^-$ bezeichnete Anion gegen ein pharmazeutisch annehmbares Anion austauschen. Auch bei dieser Austauschreaktion handelt es sich um eine an sich bekannte und jedem Fachmann geläufige Reaktion. Man verwendet vorzugsweise herkömmliche Ionenaustauscher, welche mit einem pharmazeutisch annehmbaren Anion beladen sind.

Gemäss Verfahrensvariante f) können Verbindungen der Formel I in pharmazeutisch annehmbare Säureadditionssalze übergeführt werden. Derartige Säureadditionssalze können nach an sich bekannten und jedem Fachmann geläufigen Methoden hergestellt werden. Es kommen dabei sowohl Salze mit anorganischen als auch Salze mit organischen Säuren in Betracht, beispielsweise Hydrochloride, Hydrobromide, Sulfate, Nitrate, Citrate, Acetate, Maleate, Succininate, Methansulfonate, p-Toluolsulfonate und dergleichen.

Die als Ausgangsstoffe verwendeten Verbindungen der Formel II, können beispielsweise gemäss dem nachfolgenden Reaktionsschema I hergestellt werden, worin $R^3$, $R^5$, $Ya^-$ und die gestrichelte Linie obige Bedeutung besitzen:

Reaktionsschema I

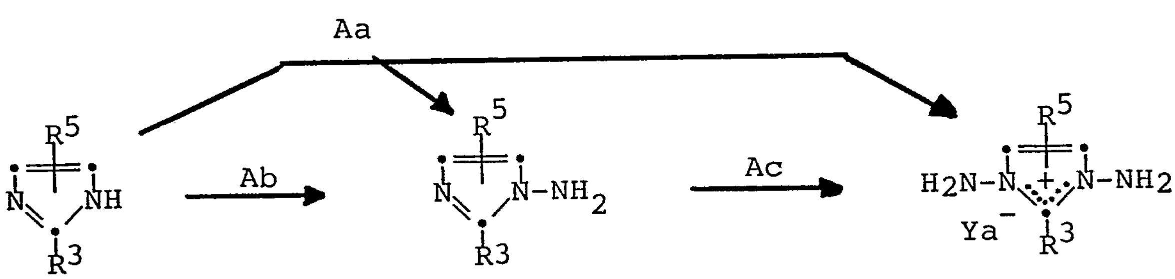


IXa Xa II

Verbindungen der Formel II, worin $R^3$ niederes Alkylthio bedeutet, können beispielsweise auch gemäss dem nachfolgenden Reaktionsschema II hergestellt werden, worin $R^5$, R", $Ya^-$ und die gestrichelte Linie obige Bedeutung besitzen, und Ø Phenyl bedeutet:

Reaktionsschema II

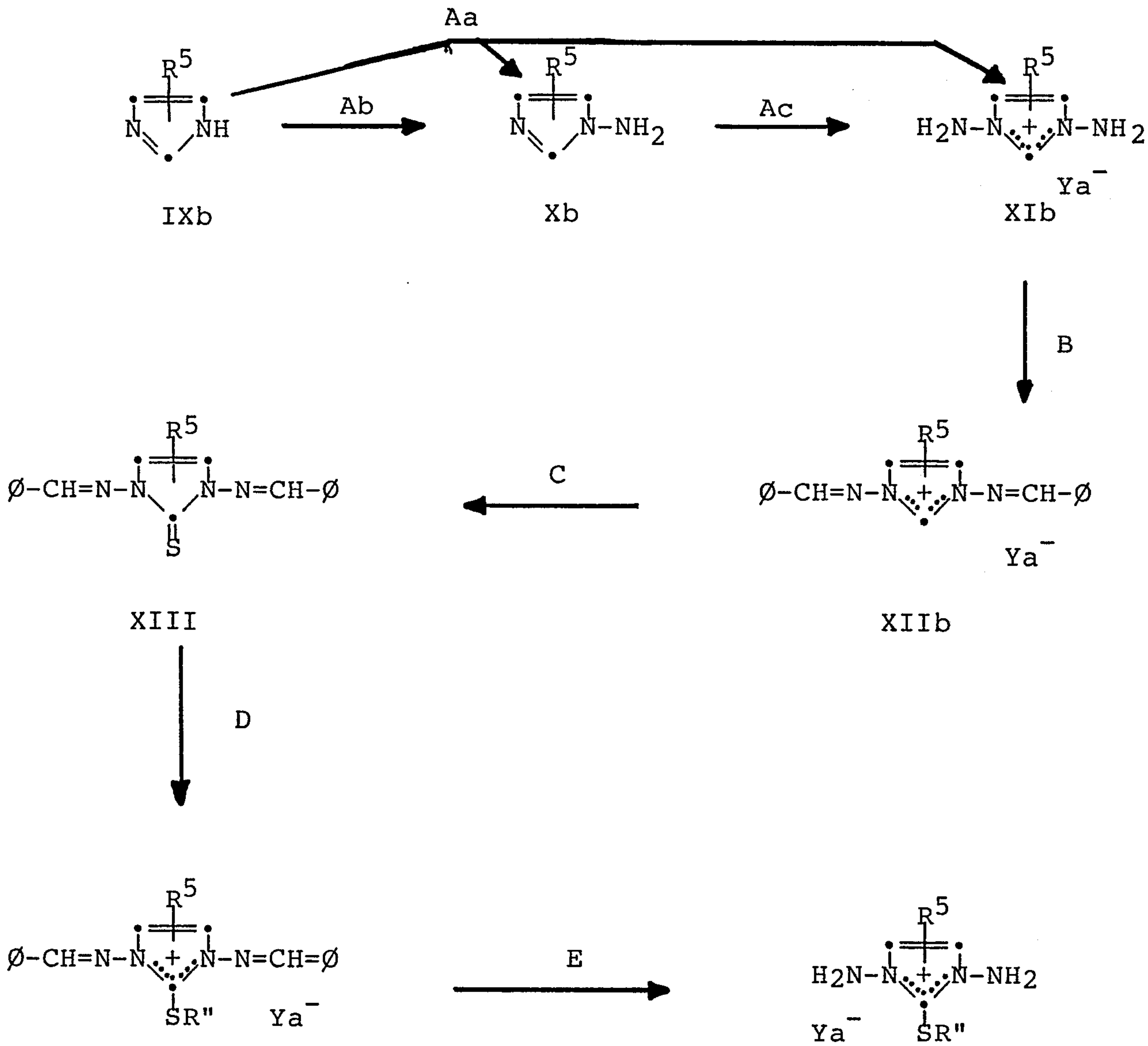


IXb Xb XIb

XIII XIIb

XIVb IIb

Verbindungen der Formel II, worin $R^3$ die Gruppe -CH=CH-Ra bedeutet, können beispielsweise auch gemäss dem nachfolgenden Reaktionsschema III hergestellt werden, worin $R^5$, Ra, $Ya^-$, die gestrichelte Linie und Ø obige Bedeutung besitzen:

Reaktionsschema III

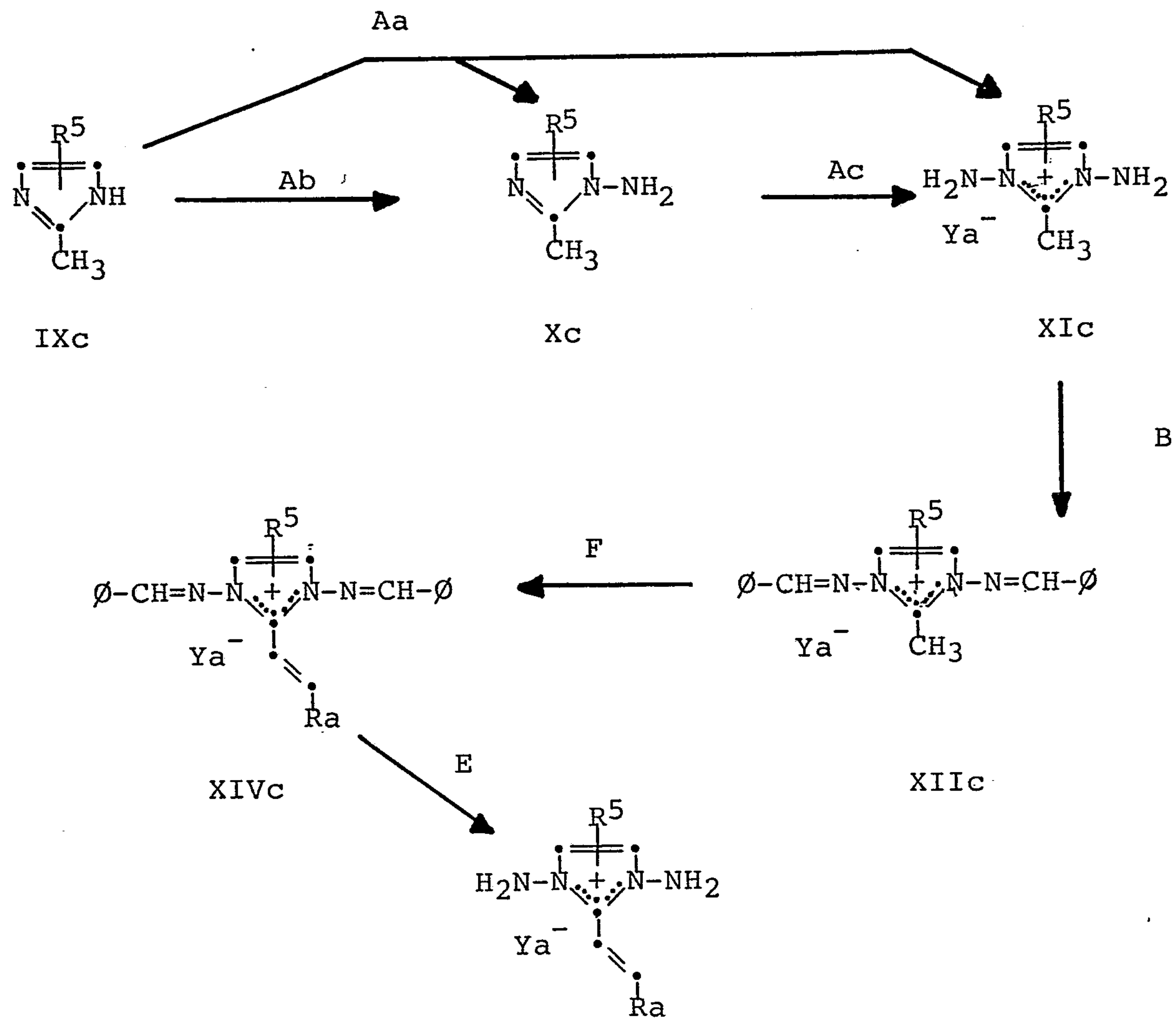


IXc Xc XIc

XIVc XIIc

IIc

Verbindungen der Formel II, worin $R^3$ eine basische Aminogruppe bedeutet, können beispielsweise auch gemäss dem nachfolgenden Reaktionsschema IV hergestellt werden, worin $R^5$, $Ya^-$, die gestrichelte Linie und Ø obige Bedeutung besitzen, und Ra" eine basische Aminogruppe bedeuten:

Reaktionsschema IV

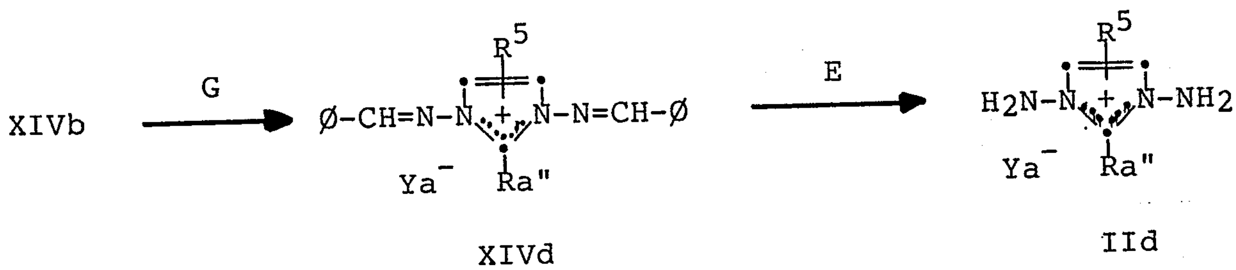


Die Verbindungen der Formel II, worin $R^3$ die Gruppe -A'-Ra", A' niederes Alkylen und Ra" eine basische Aminogruppe bedeuten, können beispielsweise auch gemäss dem nachfolgenden Reaktionsschema V hergestellt werden, worin $R^5$, Ra", A', Ø, $Ya^-$ und die gestrichelte Linie obige Bedeutung besitzen, und X' ein Halogenatom bedeutet:

Reaktionsschema V

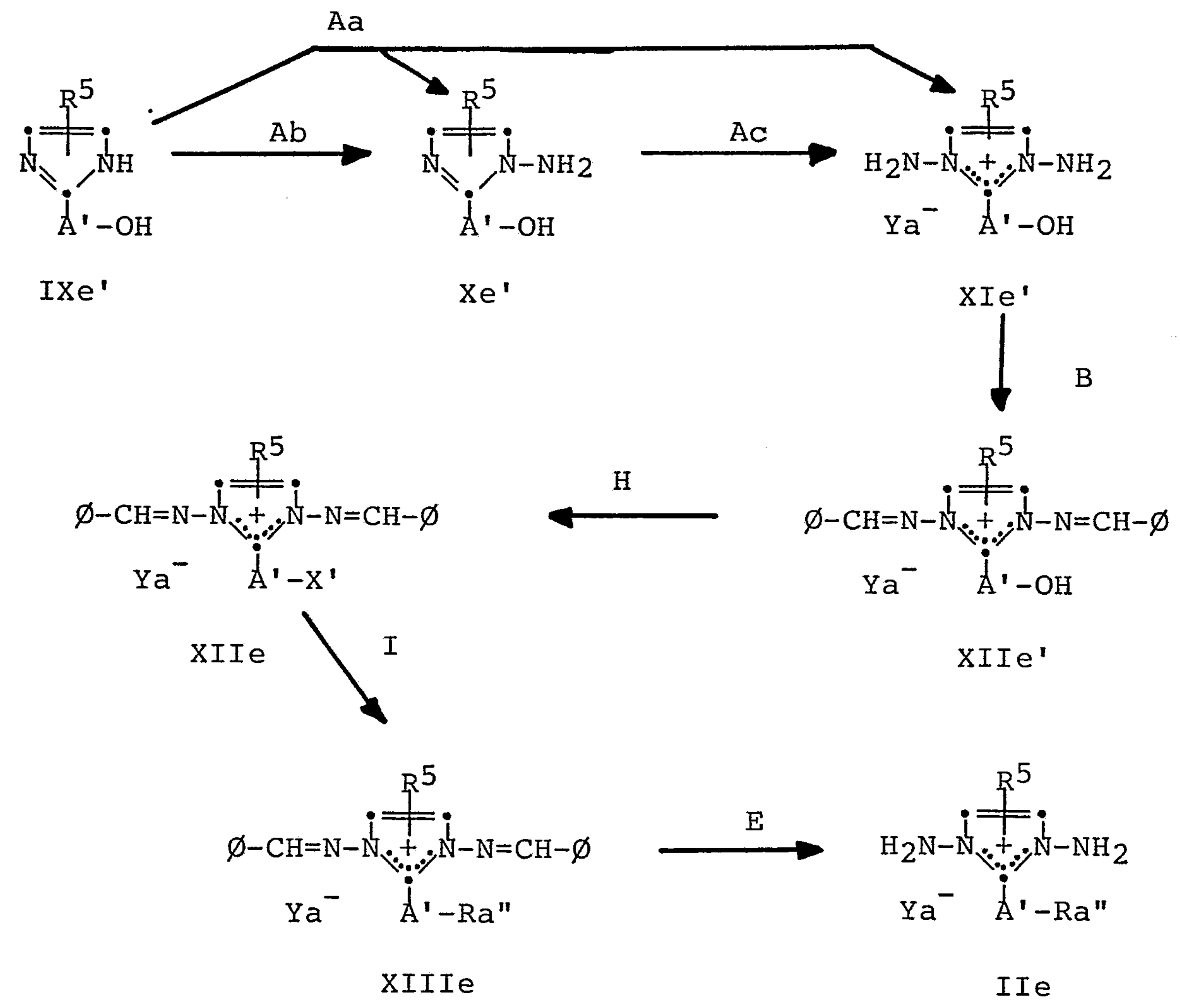


IXe' Xe' XIe' XIIe' XIIe XIIIe IIe

Die Verbindungen der Formel III können gemäss den vorstehenden Reaktionsschemata I-V hergestellt werden, indem man jeweils an Stelle einer Verbindung der Formel X eine entsprechende Verbindung der allgemeinen Formel

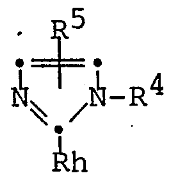

XV

worin Rh Wasserstoff, Methyl, die Gruppe -A'-OH oder $R^3$ bedeutet, und A', $R^3$, $R^4$ und $R^5$ obige Bedeutung besitzen, als Ausgangsstoff einsetzt.

Die Verbindungen der Formel III, worin $R^4$ eine gesättigte oder partiell ungesättigte niedere Kohlenwasserstoffgruppe oder die Gruppe -CHRcRd oder -CHRc-CO-Re bedeutet, können beispielsweise auch gemäss dem nachfolgenden Reaktionsschema VI hergestellt werden, worin $R^3$, $R^{41}$, $R^5$, $Ya^-$, Ø und die gestrichelte Linie obige Bedeutung besitzen:

<u>Reaktionsschema VI</u>

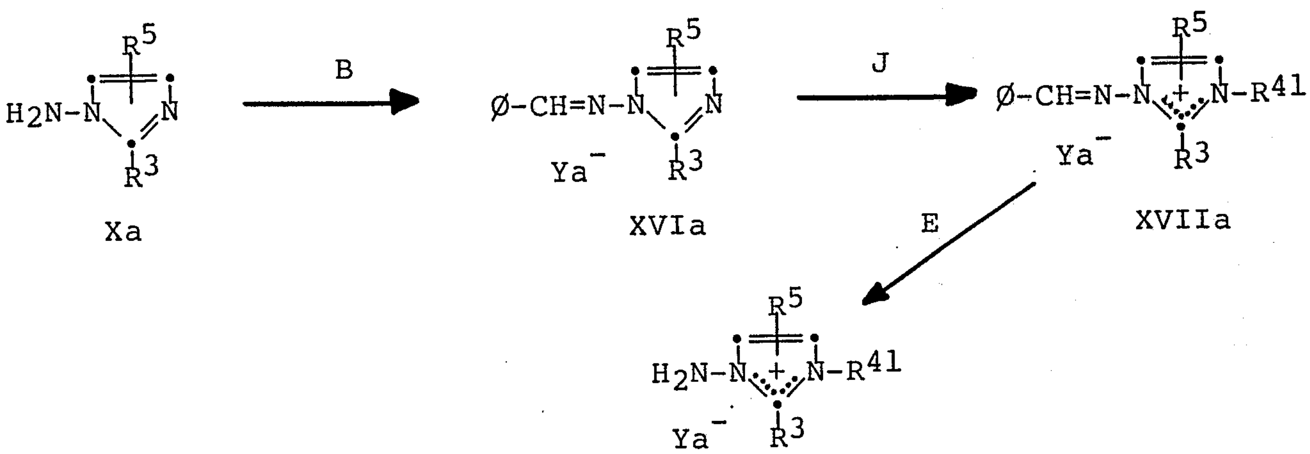


Xa XVIa XVIIa

IIIa

Die Verbindungen der Formel III, worin $R^4$ eine gesättigte oder partiell ungesättigte niedere Kohlenwasserstoffgruppe oder die Gruppe -CHRcRd oder -CHRc-CO-Re und $R^3$ die Gruppe -SR", -CH=CH-Ra, Ra" oder -A'-Ra" bedeuten, können auch hergestellt werden, indem man eine Verbindung der Formel Xb, Xc oder Xe' gemäss vorstehendem Reaktionsschema VI an Stelle der Verbindung der Formel Xa in die der Formel XVIIa entsprechende Verbindung überführt und diese an Stelle der Verbindungen der Formel XII gemäss dem Reaktionsschema II, III, IV und V weiterverarbeitet.

Nachstehend werden die in den Reaktionsschemata erwähnten Reaktionsschritte A-G näher erläutert. Es handelt sich dabei durchwegs um an sich bekannt und jedem Fachmann geläufige Reaktionen.

Aa: Bei diesem Reaktionsschritt handelt es sich um eine elektrophile Aminierung mit einem Aminierungsmittel wie O-(2,4-Dinitrophenyl)hydroxylamin, O-Mesitylensulfonylhydroxylamin oder Hydroxylamin-O-sulfonsäure oder dessen Salzen mit anorganischen Basen. Vorzugsweise verwendet man die entsprechenden Alkalimetallsalze der Hydroxylamin-O-sulfonsäure, beispielsweise das Natriumsalz, und arbeitet in wässriger Lösung. Man erhält dabei in der Regel ein Gemisch bestehend aus dem Diamin der Formel II bzw. XI und dem Monoamin der Formel X. Die beiden Verbindungen können nach an sich bekannten und jedem Fachmann geläufigen Methoden voneinander getrennt werden. Die weiter unten folgenden Beispiele enthalten detaillierte Angaben betreffend die Auftrennung der erhaltenen Gemische.

Ab: Bei diesem Reaktionsschritt handelt es sich um eine elektrophile Aminierung mit einem Aminierungsmittel wie O-Diphenylphosphinylhydroxylamin oder O-Mesitylensulfonylhydroxylamin, wobei man das Ausgangsmaterial vor der Umsetzung mit dem Aminierungsmittel mit einer starken Base in ein Alkalimetallsalz überführt. Geeignete Basen sind beispielsweise niedere Alkalimetallalkoxide, wie Natriummethoxid und Natriumäthoxid, und Alkalimetallhydride, wie Natriumhydrid.

Geeignete Lösungsmittel sind beispielsweise N-Methylpyrrolidon, N,N-Dimethylformamid, niedere Alkohole, Aether, wie Tetrahydrofuran, Diäthylenglykoldimethyläther, Diäthylenglykoldiäthyläther und Diäthylenglykoldibutyläther. Die Reaktionstemperatur liegt vorzugsweise in einem Bereich von etwa 0°C bis 100°C, wobei man vorzugsweise bei Raumtemperatur arbeitet.

Ac: Bei diesem Reaktionsschritt handelt es sich um eine elektrophile Aminierung mit einem Aminierungsmittel wie O-Diphenylphosphinylhydroxylamin oder O-Mesitylensulfonylhydroxylamin. Geeignete Lösungsmittel sind beispielsweise halogenierte niedere Kohlenwasserstoffe, wie Chloroform, Methylenchlorid und Aethylenchlorid, Aether, wie Diäthyläther und Tetrahydrofuran, niedere Alkohole, wie Aethanol, Acetonitril, N,N-Dimethylformamid, Dimethylsulfoxid und Mischungen davon. Die Reaktionstemperatur liegt in einem Bereich von etwa 0°C bis zur Siedetemperatur des gewählten Lösungsmittels. Man arbeitet vorzugsweise bei Raumtemperatur.

B: Bei diesem Reaktionsschritt wird das entsprechende Ausgangsmaterial mit Benzaldehyd zum entsprechenden Aldimin umgesetzt. Geeignete Lösungsmittel sind beispielsweise niedere Fettsäuren, wie Essigsäure und Propionsäure, niedere Alkohole, wie Methanol, Aethanol und 2-Propanol, saure wässrige Medien, halogenierte niedere Kohlenwasserstoffe, wie Methylenchlorid, Chloroform und Aethylenchlorid, offenkettige und cyclische Aether, wie Diäthyläther, Diisopropyläther, t-Butylmethyläther, Aethylenglykoldimethyläther, Diäthylenglykoldiäthyläther, Tetrahydrofuran und Dioxan, Acetonitril, N,N-Dimethylformamid und Dimethylsulfoxid. Die Reaktion kann in einem Temperaturbereich von etwa 0°C bis zur Siedetemperatur des gewählten Lösungsmittels durchgeführt werden. Man arbeitet vorzugsweise bei Raumtemperatur.

C: Die gewünschte Thioxogruppe kann beispielsweise durch Behandeln des Ausgangsstoffes mit elementarem Schwefel in

Gegenwart eines tertiären Amins und in einem geeigneten Lösungsmittel, beispielsweise in Pyridin, eingeführt werden. Die Reaktion kann in einem Bereich von etwa Raumtemperatur bis zur Siedetemperatur der Reaktionsmischung durchgeführt werden.

D: Die gewünschte Alkylierung kann beispielsweise durch Behandeln des Ausgangsstoffes mit einem Alkylierungsmittel wie einem Tri(niederen Alkyl)oxoniumtetrafluoroborat, z.B. mit Trimethyloxoniumtetrafluoroborat, bewerkstelligt werden. Als Lösungsmittel eignen sich beispielsweise halogenierte niedere Kohlenwasserstoffe, wie Methylenchlorid. Man arbeitet vorzugsweise bei Raumtemperatur.

E: Bei diesem Reaktionsschritt handelt es sich um die Hydrolyse eines Aldimins. In einer bevorzugten Ausführungsform behandelt man das entsprechende Ausgangsmaterial mit einer wässrigen Säure und entfernt den erhaltenen aromatischen Aldehyd mittels Wasserdampfdestillation. Geeignete Säuren sind beispielsweise verdünnte Salzsäure und verdünnte Bromwasserstoffsäure und Tetrafluorborsäure.

F: Bei dieser Reaktion wird eine Verbindung der Formel XIIc mit einem Aldehyd der Formel VIII kondensiert. Sofern Ra Aryl oder Heteroaryl bedeutet, wird die Reaktion vorzugsweise in Gegenwart eines sekundären Amins, insbesondere eines cyclischen Amins, wie Piperidin oder Morpholin, durchgeführt. Als Lösungsmittel eignen sich beispielsweise niedere Alkohole, wie Methanol, Aethanol, 1-Propanol und 2-Propanol, aromatische Kohlenwasserstoffe, wie Benzol und Toluol, offenkettige und cyclische Aether, wie Aethylenglykoldimethyläther, Tetrahydrofuran und Dioxan, halogenierte niedere Kohlenwasserstoffe, wie Methylenchlorid, N,N-Dimethylformamid und Dimethylsulfoxid. Man arbeitet vorzugsweise bei der Siedetemperatur des gewählten Lösungsmittels. Im einer bevorzugten Ausführungsform verwendet man als Lösungsmittel einen Schlepper, beispielsweise einen aromatischen

Kohlenwasserstoff oder einen halogenierten niederen Kohlenwasserstoff, und entfernt das gebildete Reaktionswasser mit einem Wasserabscheider.

Sofern Ra eine basische Aminogruppe bedeutet, wird der Aldehyd der Formel VIII vorzugsweise in Form eines entsprechenden Di(niederen Alkyl)acetals eingesetzt, wobei die vorerwähnten Lösungsmittel auch für den vorliegenden Fall geeignet sind. Vorzugsweise verwendet man einen niederen Alkohol, wie 1-Propanol, einen halogenierten niederen Kohlenwasserstoff, wie Methylenchlorid, oder Dimethylformamid als Lösungsmittel. Die Reationstemperatur ist nicht kritisch. Man kann in einem Bereich von etwa Raumtemperatur bis zur Siedetemperatur des gewählten Lösungsmittels arbeiten. Vorzugsweise arbeitet man jedoch in einem Bereich von etwa Raumtemperatur bis etwa 100°C.

G: Bei dieser Reaktion wird eine Verbindung der Formel XIVb mit Ammoniak oder einem primären oder sekundären basischen Amin umgesetzt. Geeignete Lösungsmittel sind beispielsweise halogenierte niedere Kohlenwasserstoffe, wie Methylenchlorid, offenkettige und cyclische Aether, wie Diäthyläther, t-Butylmethyläther, Tetrahydrofuran und Dioxan, N,N-Dimethylformamid, Acetonitril und Dimethylsulfoxid. Bevorzugte Lösungsmittel sind N,N-Dimethylformamid und Acetonitril. Die Reaktionstemperatur ist nicht kritisch, und man kann beispielsweise in einem Bereich von etwa 0°C bis zur Siedetemperatur des gewählten Lösungsmittels arbeiten. Vorzugsweise arbeitet man jedoch bei Raumtemperatur.

H: Bei dieser Reaktion wird eine Verbindung der Formel XIIe' mit einem Halogenierungsmittel wie Thionylchlorid und Phosphoroxychlorid umgesetzt, wobei man als Lösungsmittel vorzugsweise überschüssiges Halogenierungsmittel einsetzt. Die Reaktion kann in einem Temperaturbereich von etwa 0°C

bis zur Siedetemperatur der Reaktionsmischung durchgeführt werden.

I: Bei dieser Reaktion wird eine Verbindung der Formel XIIe mit Ammoniak oder einem primären oder sekundären basischen Amin umgesetzt, wobei man als Lösungsmittel vorzugsweise Aceton oder N,N-Dimethylformamid verwendet. Die Reaktion kann in einem Temperaturbereich von etwa Raumtemperatur bis zur Siedetemperatur der Reaktionsmischung durchgeführt werden.

J: Bei diesem Reaktionsschritt wird das entsprechende Ausgangsmaterial mit einer Verbindung der obigen Formel VI umgesetzt. Geeignete Lösungsmittel sind beispielsweise halogenierte niedere Kohlenwasserstoffe, wie Methylenchlorid, Chloroform und Aethylenchlorid, offenkettige und cyclische Aether, wie Diäthyläther, Diisopropyläther, Aethylenglykoldimethyläther, Diäthylenglykoldiäthyläther, Tetrahydrofuran und Dioxan, Acetonitril und N,N-Dimethylformamid. Diese Reaktion kann in einem Temperaturbereich von etwa 0°C bis zur Siedetemperatur des gewählten Lösungsmittels durchgeführt werden.

Die als Ausgangsstoffe verwendeten Verbindungen der Formel V können in Analogie zur Verfahrensvariante a) oder zum weiter oben beschriebenen Reaktionsschritt B durch Umsetzen einer Verbindung der Formel Xa mit einem Aldehyd oder Keton der Formel IV hergestellt werden.

Die als Ausgangsstoffe verwendeten Verbindungen der Formel VII und die Ausgangsstoffe zu deren Herstellung können in Analogie zu den Verfahrensvarianten a) und b) bzw. in Analogie zu den beschriebenen Verfahren für die Herstellung der in diesen Verfahrensvarianten eingesetzten Ausgangsstoffe hergestellt werden.

Die neuen, als Ausgangsstoffe verwendeten und oben

beschriebenen Verbindungen und deren Verwendung zur Herstellung von therapeutischen Wirkstoffen, welche als gemeinsames Strukturmerkmal eine Gruppe der allgemeinen Formel

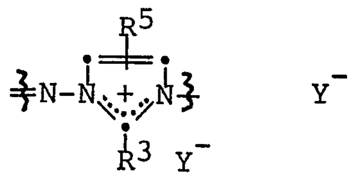

worin $R^3$, $R^5$ und $Y^-$ obige Bedeutung besitzen, aufweisen, sind ebenfalls Gegenstand der vorliegenden Erfindung. Es handelt sich dabei um die oben definierten Verbindungen der Formeln II, III und V.

Wie bereits eingangs erwähnt besitzen die Verbindungen der Formel I wertvolle pharmakologische Eigenschaften. Sie weisen beispielsweise antiparasitäre Eigenschaften auf und sind insbesondere wirksam gegen parasitäre Protozoen und Würmer. Besonders ausgeprägt ist ihre Wirkung gegen parasitäre Würmer, insbesondere gegen Nematoden wie die Filarien. Diese pharmakologischen Eigenschaften können mittels bekannter und jedem Fachmann geläufiger Testmethoden ermittelt werden.

Die filarizide Wirkung der Verbindungen der Formel I kann beispielsweise an Baumwollratten (Sigmodon hispidus) ermittelt werden, welche mit Litomosoides carinii infiziert worden sind. Die Infektion mit L. carinii wird durch die blutsaugende Milbe Bdellonyssus bacoti übertragen, in welcher sich die Entwicklung von der Mikrofilarie zur infektiösen Larve vollzieht. Die Baumwollratten werden infiziert, indem man sie dem Biss infizierter Milben aussetzt. 14 Wochen nach der Infektion werden Gruppen von 2-4 Tieren subkutan mit der zu testenden Verbindung behandelt. 42 Tage nach Behandlung mit der zu testenden Verbindung werden die Versuchstiere seziert, wobei man die addulten Filarien aus

der Pleuralhöhle entfernt. Lebende und tote oder eingekapselte Würmer werden voneinander getrennt und gewogen. Die filarizide Wirkung wird ausgedrückt als prozentualer Anteil toter Makrofilarien pro Behandlungsgruppe. Mit den Werten aus verschiedenen Dosierungsgruppen wird dann die $ED_{90}$ mittels Probitanalyse bestimmt. Die $ED_{90}$ ist diejenige Dosis, bei welcher 90% der aus der Pleuralhöhle entfernten Würmer tot sind. In der nachfolgenden Tabelle werden die Resultate zusammengestellt, welche mit repräsentativen Vertretern der durch die allgemeine Formel I definierten Verbindungsklasse in dem vorstehend geschilderten Versuch erhalten worden sind. Die Tabelle enthält ausserdem Angaben über die akute Toxizität dieser Verbindungen in mg pro kg bei einmaliger oraler Verabreichung an Mäusen.

Tabelle

| Verbindung | $ED_{90}$ in mg/kg s.c. | $DL_{50}$ in mg/kg p.o. |
|---|---|---|
| A | 1,0 | 312- 625 |
| B | 2,0 | - |
| C | 0,55 | - |
| D | <1,0 | 1250-2500 |

Verbindung A = 1,3-Bis[[p-(Dimethylamino)benzyliden]-amino]-2-[2-(dimethylamino)vinyl]imidazoliumchlorid.

Verbindung B = 1,3-Bis[[p-(Dimethylamino)benzyliden]-amino]-2-(2-piperidinovinyl)imidazoliumchlorid.

Verbindung C = 1,3-Bis[[p-(Dimethylamino)benzyliden]-amino]-2-(2-morpholinovinyl)imidazoliumchlorid.

Verbindung D = 1,3-Bis[[p-(Dimethylamino)benzyliden]-amino]-2-phenylimidazoliumchlorid.

Die Verbindungen der Formel I können als Heilmittel, z.B. in Form pharmazeutischer Präparate zur enteralen oder parenteralen Applikation, Verwendung finden. Sie können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die beschriebenen Stoffe, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele,

Wachse, Fette und halbflüssige oder flüssige Polyole.

Als pharmazeutische Hilfsstoffe kommen die üblichen Stabilisierungs-, Konservierungs-, Netz- und Emulgiermittel, Mittel zur geschmacklichen Verbesserung, Salze zur Veränderung des osmotischen Druckes, Puffersubstanzen, Lösungsvermittler, Färbe- und Ueberzugsmittel und Antioxidantien in Frage.

Die Dosierung der beschriebenen Stoffe kann, abhängig von der zu behandelnden Krankheit, dem Alter und dem individuellen Zustand des Patienten und von der Applikationsweise, innerhalb weiter Grenzen variieren und ist natürlich in jedem einzelnen Fall den individuellen Gegebenheiten anzupassen. Für die Prophylaxe und Therapie von Infektionskrankheiten, welche durch Bakterien, Pilze oder Parasiten hergerufen werden, kommt für den erwachsenen Patienten eine tägliche Dosis von etwa 0,01 g bis etwa 4 g, insbesondere etwa 0,05 g bis etwa 2 g in Betracht. Je nach Dosierung ist es dabei zweckmässig, die Tagesdosis in mehreren Dosierungseinheiten zu verabreichen.

Die erfindungsgemässen pharmazeutischen Präparate enthalten zweckmässigerweise etwa 10-1000 mg, vorzugsweise 50-500 mg eines erfindungsgemässen Stoffes.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

## Beispiel 1

a) zu einer Lösung von 7,26 g (88 mMol) 2-Methylimidazol in 30 ml Wasser wird eine bei 0° hergestellte Lösung von 10 g (88 mMol) Hydroxylamin-O-sulfonsäure und 7,4 g (88 mMol) Natriumhydrogencarbonat in 60 ml Wasser getropft. Die Temperatur steigt dabei auf 35° an. Nach Rühren während 16 Stunden wird mit 26 ml 2N-Salzsäure angesäuert. Eine Lösung von 6,26 g (59 mMol) Benzaldehyd in 20 ml Aether wird dazugegeben, worauf man noch während 6 Stunden rührt. Das ausgefallene Produkt wird abfiltriert und aus Methanol/Aether umkristallisiert. Man erhält 1,3-Bis(benzylidenamino) -2-methylimidazoliumbenzaldoxim-O-sulfonat vom Schmelzpunkt 249-250°. Das erhaltene saure Filtrat wird gemäss Beispiel 2a) weiterverarbeitet.

b) 19,2 g (39 mMol) 1,3-Bis(benzylidenamino) -2-methylimidazoliumbenzaldoxim-O-sulfonat werden in 100 ml Wasser und 70 ml 2N-Salzsäure einer Wasserdampfdestillation unterworfen, bis kein Benzaldehyd mehr entsteht. Es wird eingedampft, und der Rückstand wird auf eine mit 200 ml Ionenaustauscher Amberlite IRA 400 (Chlorid) beladene Säule gebracht. Man eluiert mit ca. 1 Liter Wasser, dampft das Eluat ein und kristallisiert das erhaltene Material aus Aethanol/-Methylenchlorid um. Man erhält 1,3-Diamino-2-methyl-imidazoliumchlorid vom Schmelzpunkt 225-227°.

c) 0,22 g (1,5 mMol) 1,3-Diamino-2-methylimidazoliumchlorid werden in 5 ml Eisessig gelöst, worauf man mit einer Lösung von 0,45 g (3 mMol) 4-Dimethylaminobenzaldehyd in 5 ml Eisessig versetzt. Nach Rühren während 2 Stunden wird der gelbe Niederschlag abfiltriert. Nach dem Trocknen erhält man 1,3-Bis[[p-(dimethylamino)benzyliden]amino] -2-methylimidazoliumchlorid vom Zersetzungspunkt 264-266°. Die Mutterlauge wird mit Aether versetzt, wobei eine zweite Portion des gewünschten Produktes erhalten wird. Das erhaltene Material wird aus heissem Wasser umkristallisiert. Man erhält 1,3-Bis[[p-(dimethylamino)benzyliden]amino] -2-methylimidazoliumchlorid vom Zersetzungspunkt 270°.

d) 0,82 g (2,0 mMol) 1,3-Bis[[p-(dimethylamino)benzyliden]-amino] -2-methylimidazoliumchlorid werden in 30 ml Aethanol suspendiert, worauf man mit 0,3 g (2,0 mMol) 4-Dimethylaminobenzaldehyd und 0,17 g (2,0 mMol) Piperidin versetzt und unter Rückfluss zum Sieden erhitzt. Dabei entsteht eine tiefrote Lösung. Nach 20 Stunden lässt man abkühlen. Das ausgefallene Produkt wird abfiltriert und aus Methylenchlorid/Aether umgefällt. Nach Umkristallisieren aus Toluol erhält man 1,3-Bis[[p-(dimethylamino)benzyliden]amino]-2-[p-(dimethylamino)styryl]imidazoliumchlorid als roten Festkörper vom Schmelzpunkt 238-240°.

## Beispiel 2

a) Das saure Filtrat aus Beispiel 1a) wird dreimal mit je 15 ml Aether ausgeschüttelt. Die wässrige Phase wird unter Kühlen mit 9 ml 4N-Natronlauge neutralisiert. Man filtriert den Niederschlag ab und fällt diesen aus Methanol/Aether um. Man erhält 1-(Benzylidenamino)-2-methylimidazol vom Schmelzpunkt 122°-124°.

b) Man löst 0,92 g (5 mMol) 1-(Benzylidenamino)-2-methylimidazol in 10 ml absolutem Dichlormethan und versetzt dann mit 0,74 g (5 mMol) Trimethyloxoniumtetrafluoroborat. Man rührt bei Raumtemperatur über Nacht, nutscht den entstandenen Niederschlag ab und wäscht diesen zweimal mit Dichlormethan. Nach Umfällen aus Acetonitril/Diäthyläther erhält man farbloses 1-(Benzylidenamino)-2,3-dimethylimidazoliumtetrafluoroborat vom Smp. 213-216°.

c) 0,86 g (3 mMol) 1-Benzylidenamino-2,3-dimethylimidazoliumtetrafluoroborat werden in 30 ml Wasser suspendiert. Es wird mit 1 ml 50-proz. Tetrafluorborsäure versetzt und so lange wasserdampfdestilliert, bis kein Benzaldehyd mehr übergeht. Die farblose, saure Lösung wird dann mit 0,45 g (3 mMol) p-(Dimethylamino)benzaldehyd versetzt und während 3 Stunden bei Raumtemperatur gerührt. Der entstandene orangegelbe Niederschlag wird abgenutscht, dreimal mit Wasser und dreimal mit Diäthyläther gewaschen und aus Methanol/Aethanol umkristallisiert. Man erhält 1-[[p-(Dimethylamino)benzyli-

den]amino] -2,3-dimethylimidazoliumtetrafluoroborat in Form von gelben Nadeln, mit einem Schmelzpunkt von 248-251°.

d) 0,66 g (2 mMol) 1-[[p-(Dimethylamino)benzyliden]amino] -2,3-dimethylimidazoliumtetrafluoroborat, 0,45 g (3 mMol) p-(Dimethylamino)benzaldehyd und 0,34 g (4 mMol) Piperidin werden in 30 ml Toluol suspendiert, worauf man während etwa 70 Stunden an einem mit Toluol, das 5% Piperidin enthält, gefüllten Wasserabscheider unter Rückfluss zum Sieden erhitzt. Das ausgefallene Material wird abgenutscht, mit Toluol, zweimal mit Diäthyläther und zweimal mit Wasser gewaschen und aus Methanol umkristallisiert. Man erhält farbloses 1-[[p-(Dimethylamino)benzyliden]amino] -2-[p-(dimethylamino)styryl] -3-methylimidazoliumtetrafluoroborat vom Schmelzpunkt 235-238°.

Beispiel 3

a) Eine Lösung von 1-(Benzylidenamino)-2-methylimidazol in 1,5N-Salzsäure wird solange einer Wasserdampfdestillation unterworfen, bis kein Benzaldehyd mehr entsteht. Es wird eingedampft, und das erhaltene Produkt wird aus Aethanol/Aether umkristallisiert. Man erhält 1-Amino-2-methylimidazol-Hydrochlorid vom Schmelzpunkt 124°.

b) 1,3 g (10 mMol) 1-Amino-2-methylimidazol-Hydrochlorid und 2,2 g (15 mMol) p-(Dimethylamino)benzaldehyd werden in 100 ml Eisessig während 16 Stunden bei Raumtemperatur gerührt. Es wird eingedampft, worauf man noch viermal mit Aethanol versetzt und jeweils wieder eindampft. Der kristalline Rückstand wird mit Aether gewaschen und dann mit gesättigter Natriumhydrogencarbonatlösung versetzt. Man extrahiert mit Methylenchlorid, trocknet den Auszug über Natriumsulfat, dampft ein und kristallisiert das erhaltene Material aus Methylenchlorid/Petroläther um. Man erhält 1-[[p-(Dimethylamino)benzyliden]amino]-2-methylimidazol vom Schmelzpunkt 166-167°.

c) 1,37 g (6 mMol) 1-[[p-(Dimethylamino)benzyliden]amino] -2-methylimidazol werden in 15 ml absolutem Dimethylformamid gelöst. Nach Zugabe von 1,57 g (6 mMol) 3,4,5-Trimethoxybenzylbromid wird über Nacht bei Raumtemperatur gerührt. Nach Kühlen des Reaktionsgemisches im Eisbad wird der entstandene Niederschlag abgenutscht, zweimal mit eiskaltem Dimethylformamid und dreimal mit Diäthyläther gewaschen und aus Acetonitril umkristallisiert. Man erhält hellgelbes 1-[[p-(Dimethylamino)benzyliden]amino] -3-(3,4,5-trimethoxy)benzyl-2-methylimidazoliumbromid von Schmelzpunkt 238-241°.

d) 1,96 g (4 mMol) 1-[[p-(Dimethylamino)benzyliden]amino] -3-(3,4,5-trimethoxy)benzyl-2-methylimidazoliumbromid, 0,9 g (6 mMol) p-(Dimethylamino)benzaldehyd und 0,7 g (8 mMol) Piperidin werden in 50 ml Toluol suspendiert, worauf man während etwa 60 Stunden am Wasserabascheider (mit Toluol, das 5% Piperidin enthält, gefüllt) unter Rückfluss zum Sieden erhitzt. Das ausgefallene Material wird abgenutscht, mit Toluol, zweimal mit Diäthyläther und danach dreimal mit Wasser gewaschen und dann aus Aethanol und Methanol umkristallisiert. Man erhält orangefarbenes 1-[[p-(Dimethylamino)benzyliden]amino] -2-[p-(dimethylamino)styryl] -3-(3,4,5-trimethoxybenzyl)imidazoliumbromid vom Schmelzpunkt 253-256° (Zersetzung).

<u>Beispiel 4</u>

a) 680 mg (0,01 Mol) Imidazol werden in einem Gemisch aus 4 ml absolutem N-Methyl-2-pyrrolidon und 1 ml absolutem Tetrahydrofuran suspendiert. Die Suspension wird auf -40° gekühlt. Unter Rühren wird innterhalb von 30 Minuten eine Lösung von 4,8 g (0,024 mMol) O-(2,4-Dinitrophenyl)hydroxylamin in 8 ml absolutem N-Methyl-2-pyrrolidon langsam zugetropft. Man lässt bei 20° während 18 Stunden stehen, versetzt das Reaktionsgemisch mit 25 ml Wasser und 12 ml 2N-Salzsäure und schüttelt fünfmal mit je 20 ml Aether aus. Die saure Lösung wird mit Aktivkohle während 15 Minuten gerührt und darauf filtriert. Nach Zugabe von 3 ml Benzaldeyd, 5 ml 2N-Salzsäure und 30 ml Aether wird drei Stunden unter Eiskühlung gerührt. Man filtriert den ausgefallenen Niederschlag ab und wäscht ihn zweimal mit Wasser und einmal mit Aether. Das so erhaltene Rohprodukt fällt man aus Methanol/Aether um. Man erhält 1,3-Bis(benzylidenamino)imidazoliumchlorid vom Schmelzpunkt 193-196°.

b) 3,11 g (0,01 Mol) 1,3-Bis(benzylidenamino)imidazoliumchlorid, 0,32 g (0,01 Mol) Schwefel und 1,01 g (0,01 Mol) Triäthylamin werden in 20 ml absolutem Pyridin suspendiert, worauf man während 15 Minuten bei Raumtemperatur rührt. Anschliessend erhitzt man das Reaktionsgemisch eine halbe Stunde lang unter Rückfluss zum Sieden. Nach Abkühlen auf Raumtemperatur versetzt man mit 100 ml Wasser, nutscht den entstandenen Niederschlag ab und wäscht dreimal mit Wasser und dreimal mit Aethanol. Man erhält hellgelbes 1,3-Bis-(benzylidenamino) -1,3-dihydro-2H-imidazol-2-thion vom Schmelzpunkt 215-218°.

c) Man versetzt 3,06 g (0,01 Mol) 1,3-Bis(benzylidenamino)--1,3-dihydro -2H-imidazol-2-thion in 100 ml absolutem Dichlormethan mit 1,47 g (0,01 Mol) Trimethyloxoniumtetrafluoroborat, rührt während 3 Stunden bei Raumtemperatur und nutscht den entstandenen Niederschlag ab. Aus dem Filtrat wird durch Zugabe von Diäthyläther eine weitere Portion des gewünschten Produktes ausgefällt, die zuammen mit der ersten Portion einmal mit Wasser, einmal mit kaltem Methanol und dreimal mit Diäthyläther gewaschen und anschliessend aus Acetonitril/Diäthyläther umgefällt wird. Man erhält farbloses 1,3-Bis(benzylidenamino)-2-(methylthio) imidazoliumtetrafluoroborat vom Schmelzpunkt 215-220° (Zersetzung).

d) 4,47 g (0,01 Mol) 1,3-Bis(benzylidenamino) -2-(methylthio)imidazoliumtetrafluoroborat werden in 100 ml Wasser suspendiert. Die Suspension wird mit 3,5 ml 50-proz. Tetrafluoroborsäure versetzt und wasserdampfdestilliert, bis kein Benzaldehyd mehr übergeht. Die auf Raumtemperatur gekühlte, farblose, saure Lösung wird mit 2,98 g (0,02 Mol) p-Dimthylaminobenzaldehyd versetzt und während 3 Stunden bei Raumtemperatur gerührt. Der entstandene gelbe Niederschlag wird abgenutscht, dreimal mit Wasser, dreimal mit Aethanol und zweimal mit Diäyhyläther gewaschen und anschliessend aus Acetonitril/Diäthyläther umgefällt. Nach Umkristallisieren aus Aethanol erhält man 1,3-Bis[[p-(dimethylamino)benzyliden]amino] -2-(methylthio)imidazoliumtetrafluoroborat als gelben Festkörper vom Schmelzpunkt 250-253° (Zersetzung).

Beispiel 5

Durch eine Mischung von 2,47 g (5 mMol) 1,3-Bis[[p-(dimethylamino)benzyliden]amino] -2-(methylthio)imidazoliumtetrafluoroborat und 30 ml absolutem Dimethylformamid leitet man solange gasförmiges Dimethylamin ein, bis eine Gewichtszunahme von 0,4 g (entspricht 1,8 Aequivalenten) erreicht ist, und rührt anschliessend bei Raumtemperatur über Nacht. Aus der entstandenen rötlichen Lösung erhält man durch Zugabe von Diäthyläther einen kristallinen Niederschlag, der abgenutscht und je zweimal mit Wasser und Aethanol gewaschen wird. Nach Umfällen aus Dimethylformamid/Diäthyläther erhält man 1,3-Bis[[p-(dimethylamino)benzyliden]amino] -2-(dimethylamino)imidazoliumtetrafluoroborat als gelben Festkörper vom Schmelzpunkt 248-252° (Zersetzung).

Beispiel 6

a) Zu 4,1 g (0,01 Mol) 1,3-Bis(benzylidenamino) -2-(methylthio)imidazoliumtetrafluoroborat in 15 ml absolutem Dimethylformamid gibt man unter Argonatmosphäre 1,45 g (0,0166 Mol) absolutes Morpholin dazu, wobei sofort Methylmercaptanentwicklung einsetzt und eine gelborange Lösung entsteht. Man rührt bis zur Beendigung der Methylmercaptanabspaltung bei Raumtemperatur (etwa 12 Stunden), versetzt dann mit 120 ml Diäthyläther und nutscht den entstandenen, leicht gelblichen Niederschlag ab. Man wäscht fünfmal mit Diäthyläther, zweimal mit wenig kaltem Aethanol und fällt aus Acetonitril/Diäthyläther um. Man erhält farbloses 1,3-Bis-(benzylidenamino) -2-morpholinoimidazoliumtetrafluoroborat vom Schmelzpunkt 242-245° (Zersetzung).

b) 2,24 g (5 mMol) 1,3-Bis(benzylidenamino) -2-morpholinoimidazoliumtetrafluoroborat werden in Wasser suspendiert, worauf man mit 2 ml 50-proz. Tetrafluoroborsäure versetzt und solange wasserdampfdestilliert, bis kein Benzaldehyd mehr übergeht. Die erhaltene farblose, saure Lösung wird mit 1,5 g (10 mMol) p-(Dimethylamino)benzaldehyd versetzt und während 3 Stunden bei Raumtemperatur gerührt. Der entstandene orange-gelbe Niederschlag wird abgenutscht, dreimal mit Wasser, dreimal mit Aethanol und dreimal mit Diäthyläther gewaschen und anschliessend aus Dimethylformamid/Diäthyläther umgefällt. Man erhält gelbes 1,3-Bis[[p-(dimethylamino)benzyliden]amino] -2-morpholinoimidazoliumtetrafluoroborat vom Schmelzpunkt 275-280° (Zersetzung).

## Beispiel 7

2,47 g (5 mMol) 1,3-Bis[[p-(dimethylamino)benzyliden]-amino] -2-(methylthio)imidazoliumtetrafluoroborat werden in 50 ml absolutem Acetonitril suspendiert, worauf man mit 7,8 g absolutem 2-Amino-5-(diäthylamino)pentan versetzt. Man rührt unter Argonatmosphäre während etwa 24 Stunden bei Raumtemperatur, filtriert die unlöslichen Bestandteile ab und versetzt das Filtrat mit 450 ml Diäthyläther. Der erhaltene, hellgelbe Niederschlag wird abgenutscht, dreimal mit Diäthyläther gewaschen und aus Methanol/Diäthyläther umkristallisiert. Man erhält hellgelbes 1,3-Bis[[p-(dimethylamino)benzyliden]amino] -2-[[4-(diäthylamino)-1-methylbutyl]amino] imidazoliumtetrafluoroborat vom Schmelzpunkt 187-191° (Zersetzung).

Beispiel 8

0,99 g (2 mMol) 1,3-Bis[[p-(dimethylamino)benzyliden]-amino] -2-(methylthio)imidazoliumtetrafluoroborat werden in 15 ml absolutem Dimethylformamid gelöst. Unter Rühren leitet man bei Raumtemperatur solange trockenen Ammoniak in die Reaktionslösung ein, bis die Methylmercaptanabspaltung beendet ist. Die Lösung wird mit Diäthyläther versetzt. Der erhaltene Niederschlag wird zweimal mit Wasser und zweimal mit kaltem Methanol gewaschen und aus Dimethylformamid/Diäthyläther umgefällt. Man erhält gelbes 2-Amino-1,3-bis[[p-(dimethylamino)benzyliden]amino] imidazoliumtetrafluoroborat mit einem Zersetzungspunkt >260°.

Beispiel 9

a) 0,18 g (4 mMol) Natriumhydrid (55-proz. Dispersion in Oel) werden mit Tetrahydrofuran gewaschen. Bei 0° wird eine Lösung von 0,34 g (2 mMol) 2-(4-Methoxyphenyl)imidazol in 8,3 ml N-Methylpyrrolidon unter Rühren zugetropft. Sobald sich kein Wasserstoff mehr bildet, wird auf Raumtemperatur erwärmt. In drei Portionen werden 0,46 g (2 mMol) O-Diphenylphosphinylhydroxylamin zugegeben. Nach 18 stündigem Rühren bei Raumtemperatur werden 8 ml Wasser zugegeben. Man rührt noch 1 Stunde, extrahiert sechsmal mit je 20 ml Dichlormethan, trocknet den Auszug über Natriumsulfat und dampft ein, wobei das N-Methylpyrrolidon im Hochvakuum entfernt wird. Das Rohprodukt wird an 5 g Kieselgel unter Eluieren mit Chloroform/Aethanol chromatographiert. Man erhält 1-Amino-2-(4-methoxyphenyl)imidazol als dunkel gefärbtes Oel.

b) 0,1 g (0,53 mMol) 1-Amino-2-(4-methoxyphenyl)imidazol werden in 10 ml Dichlormethan gelöst. Man versetzt portionenweise innert 8 Tagen mit 0,29 g (1,24 mMol) O-Diphenylphosphinylhydroxylamin, dampft ein und suspendiert den Rückstand in 10 ml Wasser. Die schwerlösliche Diphenylphosphinsäure wird abfiltriert, und das Filtrat wird auf eine mit Amberlite IRA 400 (Chlorid) beladene Säule gebracht, worauf man mit Wasser eluiert. Man erhält 1,3-Diamino-2-(4-methoxyphenyl)imidazoliumchlorid.

c) Zu einer Lösung von 90 mg (0,44 mMol) 1,3-Diamino-2-(4--methoxyphenyl)imidazoliumchlorid in 2 ml Eisessig werden 131 mg (0,88 mMol) 4-(Dimethylamino)benzaldehyd gegeben. Nach 48 Stunden Rühren bei Raumtemperatur wird die dunkelgelbe Lösung eingedampft. Der Rückstand wird auf eine mit 8 g Kieselgel beladene Säule gebracht, worauf das Produkt mit Dichlormethan/Aethanol (9:1) eluiert und aus Aethanol/Aether kristallisiert wird. Man erhält 1,3-Bis[[p-(dimethylamino)benzyliden]amino] -2-(p-methoxyphenyl)imidazoliumchlorid vom Schmelzpunkt 250°.

## Beispiel 10

a) 3,55 g (75 mMol) Natriumhydrid (55-proz. Dispersion in Oel) werden mit Tetrahydrofuran gewaschen. Bei 0° wird eine Lösung von 6,5 g (37,3 mMol) 2-(4-Methoxyphenyl)imidazol in 153 ml N-Methylpyrrolidon dazugetropft. Sobald sich kein Wasserstoff mehr bildet, werden portionenweise insgesamt 8,8 g (37,7 mMol) O-Diphenylphosphinylhydroxylamin bei Raumtemperatur dazugegeben. Nach 18 Stunden Rühren versetzt man mit 150 ml Wasser, säuert mit 80 ml 2N-Salzsäure an, rührt noch 1 Stunde bei 0° und filtriert die ausgefallene Diphenylphosphinsäure ab. Zum sauren dunkelroten Filtrat gibt man 3,85 ml (38,1 mMol) Benzaldehyd und rührt während etwa 20 Stunden. Die dunkle Lösung wird zweimal mit je 120 ml Aether gewaschen und durch Zugabe von 20 ml 4N-Natronlauge unter Eiskühlung auf pH 14 gestellt. Es wird fünfmal mit je 150 ml Dichlormethan extrahiert. Der Extrakt wird über Natriumsulfat getrocknet und eingedampft, wobei das

N-Methylpyrrolidon im Hochvakuum entfernt wird. Der Rückstand wird auf eine mit 200 g Kieselgel beladene Säule gebracht, und das Produkt wird mit Dichlormethan/Aethanol (98:2) eluiert und dann aus Aethanol kristallisiert. Man erhält 1-(Benzylidenamino)-2-(p-methoxyphenyl)imidazol vom Schmelzpunkt 155°.

b) Zu einer Lösung von 4,6 g (16,6 mMol) 1-(Benzylidenamino)-2-(p-methoxyphenyl)imidazol in 250 ml absolutem Acetonitril werden 3,73 g (20,8 mMol) 1-Brom-3,3-dimethyl-2--butanon gegeben. Nach 24 Stunden Rühren bei 60° wird die Lösung eingedampft, und der Rückstand wird aus Aethanol/Aether kristallisiert. Man erhält 1-(Benzylidenamino)-2-(p--methoxyphenyl) -3-(pivaloylmethyl)imidazoliumbromid vom Schmelzpunkt 244°.

c) 5,0 g (10,9 mMol) 1-(Benzylidenamino)-2-(p-methoxyphenyl) -3-(pivaloylmethyl)imidazoliumbromid werden in 300 ml Wasser suspendiert. Man versetzt dann mit 10 ml 48-proz. Bromwasserstoffsäure. Die Suspension wird nun solange einer Wasserdampfdestillation unterworfen, bis kein Benzaldehyd mehr entsteht. Die Lösung wird eingedampft. Der Rückstand wird mit Aethanol versetzt, und das Lösungsmittel wird abdestilliert. Man erhält 1-Amino-2-(p-methoxyphenyl) -3-(pivaloylmethyl)imidazoliumbromid als Oel.

d) Zu einer Lösung von 3,7 g (10,04 mMol) 1-Amino-2-(p--methoxyphenyl) -3-(pivaloylmethyl)imidazoliumbromid in 25 ml Eisessig werden 1,50 g (10,04 mMol) p-(Dimethylamino)-benzaldehyd gegeben. Man rührt 15 Stunden bei Raumtemperatur, gibt 20 ml Aether dazu und filtriert nach weiteren 3 Stunden das Produkt ab. Nach Umkristallisieren aus Aethanol/Aether erhält man 1-[[p-(Dimethylamino)benzyliden]amino] -2-(p-methoxyphenyl)-3-(pivaloylmethyl)imidazoliumbromid vom Schmelzpunkt 241°.

## Beispiel 11

a) 9,0 g (200 mMol) Natriumhydrid (55-proz. Dispersion in Oel) werden in 600 ml absolutem Tetrahydrofuran suspendiert. 8,5 g (49 mMol) 2-(p-Methoxyphenyl)imidazol werden innert 5 Minuten dazugegeben. Man rührt, bis kein Wasserstoff mehr freigesetzt wird (1 Stunde). Bei 10° gibt man 34,5 g (148 mMol) O-Diphenylphosphinylhydroxylamin portionenweise dazu und rührt noch 40 Minuten bei 10°, dann 18 Stunden bei Raumtemperatur. Bei 5° wird gesättigte Kochsalzlösung dazugegeben, und anschliessend wird mit Methylenchlorid/Methanol (99,5:0,5) extrahiert. Der Auszug wird über Natriumsulfat getrocknet und eingedampft. Man erhält 1-Amino-2-(p-methoxyphenyl)imidazol als braunes Oel.

b) 9 g 1-Amino-2-(p-methoxyphenyl)imidazol werden in 300 ml Eisessig gelöst, worauf man mit 21,3 g (143 mMol) p-(Dimethylamino)benzaldehyd versetzt. Nach 24 Stunden wird eingedampft, und der Rückstand wird auf eine mit 300 g Kieselgel beladene Säule gebracht. Das Produkt wird mit Dichlormethan/Methanol (95:5) eluiert. Das dunkle Eluat wird mit Aktivkohle behandelt und eingedampft. Das Produkt wird zweimal aus Methylenchlorid/Petroläther kristallisiert. Man erhält 1-[[p-(Dimethylamino)benzyliden]amino] -2-p-methoxyphenyl)imidazoliumacetat vom Schmelzpunkt 93°. Man versetzt das Salz mit wässriger Natriumbicarbonatlösung und extrahiert mit Dichlormethan. Der Auszug wird über Natriumsulfat getrocknet und eingedampft. Das Produkt wird aus Petroläther kristallisiert. Man erhält 1-[[p-(Dimethylamino)benzyliden]-amino] -2-(p-methoxyphenyl)imidazol vom Schmelzpunkt 108°.

In analoger Weise erhält man:

Aus 2-(p-Tolyl)imidazol das 1-[[p-(Dimethylamino)benzyliden]amino]-2-(p-tolyl)imidazol vom Schmelzpunkt 127-128° und

aus 2-[(p-Methylthio)phenyl]imidazol das 1-[[p-(Dimethylamino)benzyliden]amino] -2-[p-(methylthio)phenyl]imidazol

vom Schmelzpunkt 147°.

c) In Analogie zu Beispiel 11b) erhält man aus 1-Amino-2--(p-methoxyphenyl)imidazol und 4-(Dimethylamino) -3,5-dimethoxybenzaldehyd das 1-[[4-(Dimethylamino)-3,5-dimethoxy-benzyliden]amino] -2-(p-methoxyphenyl)imidazol vom Schmelzpunkt 118-119° (Aether/Petroläther).

d) 0,45 g (10 mMol) Natriumhydrid (55-proz. Dispersion in Oel) wird mit absolutem Tetrahydrofuran gewaschen. Eine Lösung von 1,02 g (5 mMol) 2-(3,4-Dimethoxyphenyl)imidazol in 50 ml N-Methylpyrrolidon wird dann dazugetropft. Es wird 20 Minuten bei Raumtemperatur gerührt. Bei 0° gibt man 2,33 g (10 mMol) O-Diphenylphosphinylhydroxylamin dazu und schüttelt das Gemisch. Nach 24 Stunden Stehen wird Wasser dazugegeben und mit Methylenchlorid extrahiert. Der Auszug wird über Natriumsulfat getrocknet und eingedampft. Das verbleibende Oel (1-Amino-2-(3,4-dimethoxyphenyl)imidazol) wird in 20 ml Eisessig gelöst. Dazu gibt man 1,49 g (10 mMol) p-(Dimethylamino)benzaldehyd. Man lässt 2 Tage bei Raumtemperatur stehen und dampft ein. Das Rohprodukt wird auf eine mit 100 g Kieselgel beladene Säule gebracht. Das Produkt wird mit Mehylenchlorid/Methanol (19:1) eluiert. Das Eluat wird mit Natriumbicarbonatlösung versetzt und mit Methylenchlorid extrahiert. Der Extrakt wird über Natriumsulfat getrocknet und eingedampft. Nach Kristallisieren aus Aether/Petroläther erhält man 1-[[p-(Dimethylamino)benzyliden]amino] -2-(3,4-dimethoxyphenyl)imidazol vom Schmelzpunkt 149°.

In analoger Weise erhält man:

Aus 2-Phenylimidazol das 1-[[p-(Dimethylamino)benzyliden]amino] -2-phenylimidazol vom Schmelzpunkt 137°;

aus 2-(p-Aethoxyphenyl)imidazol das 1-[[p-(Dimethylamino)benzyliden]amino] -2-(p-äthoxyphenyl)imidazol vom Schmelzpunkt 168-169°;

aus 2-(m-Methoxyphenyl)imidazol das 1-[[p-(Dimethylamino)benzyliden]amino] -2-(m-methoxyphenyl)imidazol vom Schmelzpunkt 131-133° (Methylenchlorid/Petroläther);

aus 2-(3,4,5-Trimethoxyphenyl)imidazol das 1-[[p-(Dimethylamino)benzyliden]amino] -2-(3,4,5-trimethoxyphenyl)imidazol vom Schmelzpunkt 163° (Aethanol) und

aus 2-(p-Nitrophenyl)imidazol das 1-[[p-(Dimethylamino)-benzyliden]amino] -2-(p-nitrophenyl)imidazol vom Schmelzpunkt 200° (Aethanol/Aether).

e) Eine Lösung von 160 mg (0,5 mMol) 1-[[p-(Dimethylamino)-benzyliden]amino] -2-(p-methoxyphenyl)imidazol und 400 mg (2,2 mMol) 1-Brom-3,3-dimethyl-2-butanon in 3 ml Acetonitril wird 20 Stunden bei 60° gerührt. Das Produkt wird abfiltriert und mit Aether gewaschen. Man erhält 1-[[p-(Dimethylamino)benzyliden]amino] -2-(p-methoxyphenyl)-3-(pivaloylmethyl)imidazoliumbromid vom Schmelzpunkt 262°.

In analoger Weise erhält man:

f) Aus 1-[[p-(Dimethylamino)benzyliden]amino]-2-phenylimidazol und 1-Brom-3,3-dimethyl-2-butanon nach 2 Tagen bei 60° das 3-[[p-(Dimethylamino)benzyliden]amino] -2-phenyl-1--(pivaloylmethyl)imidazoliumbromid vom Schmelzpunkt 245°;

g) aus 1-[[p-(Dimethylamino)benzyliden]amino]-2-(p-tolyl)-imidazol und 1-Brom-3,3-dimethyl-2-butanon nach 20 Stunden bei 60° das 3-[[p-(Dimethylamino)benzyliden]amino] -1-(pivaloylmethyl)-2-(p-tolyl)imidazoliumbromid vom Schmelzpunkt 260°;

h) aus 1-[[p-(Dimethylamino)benzyliden]amino]-2-(3,4-dimethoxyphenyl)imidazol und 1-Brom-3,3-dimethyl -2-butanon nach 20 Stunden bei 60° das 3-[[p-(Dimethylamino)benzyliden]amino]-2 -(3,4-dimethoxyphenyl)-1-(pivaloylmethyl)imidazoliumbromid vom Schmelzpunkt 270-271°;

i) aus 1-[[p-(Dimethylamino)benzyliden]amino]-2-[p-(methylthio)phenyl]imidazol und 1-Brom-3,3-dimethyl -2-butanon nach 4 Tagen bei 60° das 3-[[p-(Dimethylamino)benzyliden]amino] -2-[p-(methylthio)phenyl]-1-(pivaloylmethyl)imidazoliumbromid vom Schmelzpunkt 255°;

j) aus 1-[[p-(Dimethylamino)benzyliden]amino]-2-(p-äthoxyphenyl)imidazol und 1-Brom-3,3-dimethyl -2-butanon nach 4 Tagen bei 60° das 3-[[p-(Dimethylamino)benzyliden]amino] -2-(p-äthoxyphenyl)-1-(pivaloylmethyl)imidazoliumbromid vom Schmelzpunkt 242°;

k) aus 1-[[p-(Dimethylamino)benzyliden]amino]-2-(3,4,5-trimethoxyphenyl)imidazol und 1-Brom-3,3-dimethyl -2-butanon das 3-[[p-(Dimethylamino)benzyliden]amino] -2-(3,4,5-trimethoxyphenyl)-1-(pivaloylmethyl) imidazoliumbromid vom Schmelzpunkt 224° (Aethanol/Aether);

l) aus 1-[[p-(Dimethylamino)benzyliden]amino]-2-(m-methoxyphenyl)imidazol und 1-Brom-3,3-dimethyl -2-butanon das 3-[[p-(Dimethylamino)benzyliden]amino] -2-(m-methoxyphenyl)--1-(pivaloylmethyl)imidazoliumbromid vom Schmelzpunkt 238° (Methylenchlorid/Methanol/Petroläther; Zersetzung);

m) aus 1-[[p-(Dimethylamino)benzyliden]amino]-2-(p-nitrophenyl)imidazol und 1-Brom-3,3-dimethyl -2-butanon das 3-[[p-(Dimethylamino)benzyliden]amino] -2-(p-nitrophenyl) -1-(pivaloylmethyl)imidazoliumbromid vom Schmelzpunkt 258° (Aethanol/Aether/Hexan) und

n) aus 1-[[4-(Dimethylamino)-3,5-dimethoxybenzyliden]-amino] -2-(p-methoxyphenyl)imidazol und 1-Brom-3,3-dimethyl -2-butanon nach 15 Tagen bei Raumtemperatur das 3-[[4-(Dimethylamino)-3,5-dimethoxybenzyliden]amino] -2-(p-methoxyphenyl)-1-(pivaloylmethyl)imidazoliumbromid vom Schmelzpunkt 218-220° (Acetonitril/Aether).

## Beispiel 12

a) Zu einer Lösung von 0,80 g (2,5 mMol) 1-[[p-(Dimethylamino)benzyliden]amino] -2-(p-methoxyphenyl)imidazol in 80 ml Acetonitril werden 0,60 g (2,5 mMol) p-(Dimethylamino)phenacylbromid gegeben. Nach 3 Tagen Rühren bei Raumtemperatur wird das Produkt abfiltriert und mit Aether gewaschen. Man erhält 3-[[p-(Dimethylamino)benzyliden]amino] -1-[p-(dimethylamino)phenacyl] -2-(p-methoxyphenyl)imidazoliumbromid vom Schmelzpunkt 234°.

b) In analoger Weise erhält man aus 1-[[p-(Dimethylamino)-benzyliden]amino] -2-(3,4,5-trimethoxyphenyl)imidazol nach 2 Tagen bei 50° in Acetonitril das 3-[[p-(Dimethylamino)-benzyliden]amino] -1-[p-(dimethylamino)phenacyl] -2-(3,4,5--trimethoxyphenyl)imidazoliumbromid vom Schmelzpunkt 232° (Aethanol/Aether).

## Beispiel 13

Zu einer Lösung von 0,32 g (1 mMol) 1-[[p-(Dimethylamino)benzyliden]amino] -2-(p-methoxyphenyl)imidazol in 20 ml 2-Propanol werden 0,92 g (4 mMol) p-Methoxyphenacylbromid gegeben. Nach 3 Tagen Rühren unter Lichtausschluss wird eingedampft. Der Rückstand wird auf eine mit 20 g Kieselgel beladene Säule gebracht. Das Produkt wird mit Methylenchlorid/Methanol (9:1) eluiert. Nach Kristallisieren aus Aethanol/Aether erhält man 3-[[p-(Dimethylamino)benzyliden]amino] -2-(p-methoxyphenyl)-1-(p-methoxyphenacyl)imidazoliumbromid vom Schmelzpunkt 188-191°.

## Beispiel 14

a) Zu einer Lösung von 0,64 g (2 mMol) 1-[[p-(Dimethylamino)benzyliden]amino] -2-(p-methoxyphenyl)imidazol in 20 ml Acetonitril werden 0,93 g p-Chlorphenacylbromid gegeben. Nach 6 Stunden Rühren bei 60° lässt man das Gemisch

16 Stunden stehen, filtriert das Produkt ab und wäscht es mit Aether. Man erhält 1-(p-Chlorphenacyl)-3-[[p-(dimethylamino)benzyliden]amino] -2-(p-methoxyphenyl)imidazoliumbromid vom Schmelzpunkt 242°.

In analoger Weise erhält man aus 1-[[p-(Dimethylamino)-benzyliden]amino] -2-(p-methoxyphenyl)imidazol und:

b) p-Nitrophenacylbromid nach 3 Tagen bei Raumtemperatur das 3-[[p-(Dimethylamino)benzyliden]amino] -2-(p-methoxyphenyl)-1-(p-nitrophenacyl)imidazoliumbromid von Schmelzpunkt 232°;

c) Aethyl-2-bromacetat nach 2 Tagen bei 50° das 1-[[p-(Dimethylamino)benzyliden]amino] -3-((äthoxycarbonyl)methyl] -2-(p-methoxyphenyl)imidazoliumbromid vom Schmelzpunkt 165-167°;

d) 3,4,5-Trimethoxybenzylchlorid nach 2 Tagen bei 60° das 3-[[p-(Dimethylamino)benzyliden]amino] -2-(p-methoxyphenyl)--1-(3,4,5-trimethoxybenzyl) imidazoliumchlorid vom Schmelzpunkt 227-228° (Aethanol/Aether);

e) t-Butyl-2-bromacetat nach 2 Tagen bei Raumtemperatur das 1-[(t-Butoxycarbonyl)methyl] -3-[[p-(dimethylamino)benzyliden]amino] -2-(p-methoxyphenyl)imidazoliumbromid vom Schmelzpunkt 203° (Acetonitril).

## Beispiel 15

a) 2,05 g (5 mMol) 1,3-Bis[[p-(dimethylamino)benzyliden]-amino] -2-methylimidazoliumchlorid und 7,35 g (50 mMol) N,N-Dimethylformamiddiäthylacetal werden in 150 ml 1-Propanol während 45 Minuten bei 100° gerührt. Durch Zugabe von Aether wird das Produkt ausgefällt. Es wird aus Dichlormethan/Methanol kristallisiert und aus Aethanol umkristallisiert. Man erhält 1,3-Bis[[p-(dimethylamino)benzyliden]-

amino] -2-[2-(dimethylamino)vinyl]imidazoliumchlorid vom Schmelzpunkt 246-248°.

In analoger Weise erhält man aus 1,3-Bis[[p-(dimethylamino)benzyliden]amino] -2-methylimidazoliumchlorid und:

b) Tetrahydro-4H-1,4-thiazin-4-carboxaldehyd-diäthylacetal nach 7 Tagen bei 55° in Dimethylformamid das 1,3-Bis[[p--(dimethylamino)benzyliden]amino] -2-[2-(tetrahydro-4H-1,4--thiazin-4-yl)vinyl]imidazoliumchloridvom Schmelzpunkt 247° (Zersetzung);

c) cis-2,6-Dimethylmorpholin-4-carboxaldehyd-diäthylacetal nach 6 Tagen bei 50° in Dimethylformamid das 1,3-Bis[[p--(dimethylamino)benzyliden]amino] -2-[2-(cis-2,6-dimethylmorpholino)vinyl]imidazoliumchlorid vom Schmelzpunkt 251° (Zersetzung);

d) Pyrrolidin-1-carboxaldehyd-diäthylacetal nach 24 Stunden bei Rückflusstemperatur in Methylenchlorid das 1,3-Bis[[p--(dimethylamino)benzyliden]amino] -2-[2-(1-pyrrolidinyl)-vinyl]imidazoliumchlorid vom Schmelzpunkt 260° (Zersetzung);

e) Morpholin-4-carboxaldehyd-diäthylacetal nach 4 Tagen bei Rückflusstemperatur in Methylenchlorid das 1,3-Bis[[p--(dimethylamino)benzyliden]amino] -2-(2-morpholinovinyl)imidazoliumchlorid vom Schmelzpunkt 259-260° (Zersetzung);

f) Piperidin-1-carboxaldehyd-diäthylacetal nach 4 Tagen bei Rückflusstemperatur in Methylenchlorid das 1,3-Bis[[p--(dimethylamino)benzyliden]amino] -2-(2-piperidinovinyl)imidazoliumchlorid vom Schmelzpunkt 240° (Zersetzung);

g) 4-Methylpiperazin-1-carboxaldehyd-diäthylacetal nach 6 Tagen bei 60° in Dimethylformamid das 1,3-Bis[[p-(dimethylamino)benzyliden]amino] -2-[2-(4-methyl -1-piperazinyl)-vinyl]imidazoliumchlorid vom Schmelzpunkt 256-258° (Zersetzung).

## Beispiel 16

a) In Analogie zu Beispiel 1 c) erhält man aus 1,3-Diamino-2-methylimidazoliumchlorid und 2,6-Dimethyl-4-(dimethylamino)benzaldehyd das 1,3-Bis[[4-(dimethylamino) -2,6-dimethylbenzyliden]amino] -2-methylimidazoliumchlorid vom Schmelzpunkt 216°.

b) In Analogie zu Beispiel 15 a) erhält man aus 1,3-Bis[[4-(dimethylamino) -2,6-dimethylbenzyliden]amino] -2-methylimidazoliumchlorid und N,N-Dimethylformamid-diäthylacetal nach 4 Stunden bei Raumtemperatur in 1-Propanol das 1,3-Bis[[4-(dimethylamino) -2,6-dimethylbenzyliden]amino] -2-[2-dimethylamino)vinyl]imidazoliumchlorid vom Schmelzpunkt 230-235° (Zersetzung).

## Beispiel 17

73,5 g (0,5 Mol) N,N-Dimethylformamiddiäthylacetal werden zu 20,5 g (0,05 Mol) 1,3-Bis[[p-(dimethylamino)-benzyliden]amino] -2-methylimidazoliumchlorid in 400 ml trockenem Dimethylformamid gegeben. Nach Rühren während 4 Tagen bei 50° lässt man abkühlen, gibt 250 ml Aether dazu und filtriert das Produkt ab. Es wird aus trockenem Aethanol umkristallisiert. Man erhält 1,3-Bis[[p-(dimethylamino)-benzyliden]amino] -2-[2-(dimethylamino)vinyl]imidazoliumchlorid vom Schmelzpunkt 265° (Zersetzung).

## Beispiel 18

a) 32,8 g (0,75 Mol) Natriumhydrid (55-proz. Dispersion in Oel) werden mit absolutem Tetrahydrofuran gewaschen. Eine Lösung von 53,1 g (0,37 Mol) 2-Phenylimidazol in 1500 ml N-Methylpyrrolidon wird dann bei 0° dazugetropft. Man rührt eine halbe Stunde bei 0° und etwa während 1,5 Stunden bei

Raumtemperatur (bis keine Gasentwicklung mehr zu beobachten ist). 85 g (0,36 Mol) O-Diphenylphosphinylhydroxylamin werden nun dazugegeben. Man rührt das Gemisch während 20 Stunden bei Raumtemperatur und gibt anschliessend 910 ml Wasser dazu. Die dunkle Lösung wird 1 Stunde bei Raumtemperatur gerührt und dann siebenmal mit je 300 ml Dichlormethan extrahiert. Der Auszug wird über Natriumsulfat getrocknet und eingedampft. Das verbleibende Oel wird auf eine mit 900 g Kieselgel beladene Säule gebracht, worauf man mit Dichlormethan/Aethanol (85:15) das 1-Amino-2-phenylimiazol eluiert.

In analoger Weise erhält man aus 2-(p-Chlorphenyl)imidazol das 1-Amino-2-(p-chlorphenyl)imidazol vom Schmelzpunkt 160° (Aethanol/Petroläther).

b) Zu 30,1 g (0,188 Mol) 1-Amino-2-phenylimidazol in 2000 ml Dichlormethan werden 43,8 g (0,188 Mol) O-Diphenylphosphinylhydroxylamin gegeben. Nach Rühren während 3 Tagen bei Raumtemperatur werden nochmals 21,9 g (0,094 Mol) O-Diphenylphosphinylhydroxylamin zugefügt. Nach einem Tag wird das Gemisch filtriert, und das Filtergut wird viermal mit je 150 ml Dichlormethan gewaschen. Filtrat und Waschlösung werden eingedampft. Der Rückstand wird auf eine mit 700 ml Ionenaustauscher Amberlite IRA 400 (Chlorid) beladene Säule gebracht, worauf man mit Wasser eluiert. Das Eluat wird eingedampft, und der Rückstand wird zweimal mit Aethanol versetzt und eingedampft. Nach Trocknen im Hochvakuum über Kaliumhydroxid erhält man 1,3-Diamino-2-phenylimidazoliumchlorid.

In analoger Weise erhält man aus 1-Amino-2-(p-chlorphenyl)imidazol das 1,3-Diamino-2-(p-chlorphenyl)imidazoliumchlorid vom Schmelzpunkt 210° (Aethanol/Aether; enthält 7% Ammoniumchlorid und 1% Wasser).

c) 0,45 g (2,1 mMol) 1,3-Diamino-2-phenylimidazoliumchlorid werden in 6 ml Eisessig gelöst. 0,64 g (4,3 mMol) 4-Dimethylaminobenzaldehyd werden dazugegeben. Nach Rühren während 24 Stunden wird etwa 1 ml absoluter Aether dazugegeben. Ein Tag später wird eingedampft, und der Rückstand wird auf eine mit 25 g Kieselgel beladene Säule gebracht. Nach Eluieren mit Dichlormethan/Aethanol (98:2) und Kristallisieren aus Aethanol/Aether erhält man 1,3-Bis[[p-(dimethylamino)-benzyliden]amino] -2-phenylimidazoliumchlorid vom Schmelzpunkt 242°.

d) In analoger Weise erhält man aus 1,3-Diamino-2-(p-chlorphenyl)imidazoliumchlorid das 2-(p-Chlorphenyl)-1,3-bis[[p--(dimethylamino) benzyliden]amino]imiazoliumchlorid vom Schmelzpunkt 242° (Zersetzung).

Beispiel 19

a) 0,88 g (20 mMol) Natriumhydrid (55-proz. Dispersion in Oel) werden mit Tetrahydrofuran gewaschen. Eine Lösung von 1,72 g (10 mMol) 2-(2-Phenyläthyl)imidazol in 50 ml N-Methylpyrrolidon wird dann bei 0° dazugetropft. Anschliessend wird bei Raumtemperatur weitergerührt, bis keine Gasentwicklung mehr zu beobachten ist. Anschliessend werden 4,66 g (20 mMol) O-Diphenylphosphinylhydroxylamin dazugegeben. Nach Rühren während 18 Stunden werden bei 0° 25 ml Wasser dazugetropft, worauf man während 1 Stunde bei Raumtemperatur weiterrührt. Nach Zugabe von 2 g Kochsalz wird mit Dichlormethan extrahiert. Der Extrakt wird über Natriumsulfat getrocknet un eingedampft. Der Rückstand wird in Methylenchlorid/Methanol/Aether aufgenommen, worauf man filtriert und eindampft. Man erhält ein Oel, welches 1-Amino-2-(2-phenyläthyl)imidazol enthält.

b) Dieses Oel (2,7 g) wird mit 50 ml Eisessig und 2,98 g (20 mMol) p-Dimethylaminobenzaldehyd versetzt. Nach Stehenlassen bei Raumtemperatur während 18 Stunden wird die Reaktionslösung eingedampft. Der Rückstand wird auf eine mit 200 g Kieselgel beladene Säule gebracht. Das Produkt wird mit Essigsäuremethylester eluiert und aus Dichlormethan/Methanol/Petroläther kristallisiert. Man erhält 1-[[p-(Dimethylamino)benzyliden]amino] -2-(phenyläthyl)imidazol vom Schmelzpunkt 153-154°.

c) Zu 0,64 g (2 mMol) 1-[[p-(Dimethylamino)benzyliden]amino] -2-(phenyläthyl)imidazol in 60 ml Acetonitril werden 8,0 g (45 mMol) 1-Brom-3,3-dimethyl-butan-2-on gegeben. Nach Rühren während 24 Stunden wird das Produkt abfiltriert. Man erhält 3-[p-(Dimethylamino)benzyliden]amino] -2-(phenyläthyl)-1-(pivaloylmethyl)imidazoliumbromid vom Schmelzpunkt 231°.

## Beispiel A

1,3-Bis[[(p-dimethylamino)benzyliden]amino] -2-[2-(dimethylamino)vinyl]imidazoliumchlorid wird in an sich bekannter Weise als Wirkstoff zur Herstellung von Tabletten nachfolgender Zusammensetzung verwendet:

| | mg/Tablette |
|---|---|
| Wirkstoff | 100 |
| Lactose | 192 |
| Maisstärke | 80 |
| Hydrolysierte Maisstärke | 20 |
| Calciumstearat | 8 |
| Tablettengewicht | 400 mg |

Als Wirkstoffe können auch die nachfolgend aufgeführten Verbindungen verwendet werden:

1,3-Bis[[(p-dimethylamino)benzyliden]amino]-2-(2-piperidinovinyl)imidazoliumchlorid;

1,3-Bis[[(p-dimethylamino)benzyliden]amino]-2-(2-morpholinovinyl)imidazoliumchlorid;

1,3-Bis[[(p-dimethylamino)benzyliden]amino]-2-phenylimidazoliumchlorid;

1,3-Bis[[(p-dimethylamino)benzyliden]amino]-2-(p-chlorphenyl)imidazoliumchlorid;

3-[[p-(Dimethylamino)benzyliden]amino]-1-(pivaloylmethyl)-2-(p-tolyl)imidazoliumbromid und

3-[[p-(Dimethylamino)benzyliden]amino]-1-(pivaloylmethyl)-2-(m-methoxyphenyl)imidazoliumbromid.

0253084

## Patentansprüche

1. Verbindungen der allgemeinen Formel

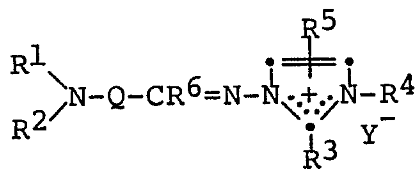

I

worin das Symbol Q Arylen oder Heteroarylen, die Gruppe $-NR^1R^2$ eine basische Aminogruppe, $R^3$ niederes Alkylthio, niederes Alkoxy oder die Gruppe $-(A)_n-Ra$, $R^4$ eine gesättigte oder partiell ungesättigte niedere Kohlenwasserstoffgruppe, eine basische Aminogruppe oder die Gruppe -N=CRc-Rb, -CHRcRd, -NH-CHRcRd, -NH-CO-Re oder -CHRc-CO-Re, $R^5$ Wasserstoff, niederes Alkyl, niederes Hydroxyalkyl, niederes Alkoxyalkyl, niederes Halogenalkyl, Aryl oder einen ankondensierten Benzolring, $R^6$ Wasserstoff oder niederes Alkyl, Ra und Rb je Aryl, Heteroaryl oder eine basische Aminogruppe, Rc Wasserstoff oder niederes Alkyl, Rd Aryl oder Heteroaryl, Re Wasserstoff, eine gegebenenfalls, über ein Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl- oder basische Aminogruppe, A Vinylen oder niederes Alkylen, n die Zahl 0 oder 1, die gestrichelte Linie eine zusätzliche Doppelbindung und das Symbol $Y^-$ ein pharmazeutisch annehmbares Anion bedeuten,
und pharmazeutisch annehmbare Säureadditionssalze davon.

2. Verbindungen gemäss Anspruch 1, worin $R^3$ niederes Alkylthio bedeutet.

3. Verbindungen gemäss Anspruch 1, worin $R^3$ die Gruppe $-(A)_n-Ra$ bedeutet.

4. Verbindungen gemäss Anspruch 3, worin n die Zahl 0 bedeutet.

5. Verbindungen gemäss Anspruch 3, worin n die Zahl 1 bedeutet.

6. Verbindungen gemäss Anspruch 5, worin A Vinylen bedeutet.

7. Verbindungen gemäss einem der Ansprüche 3-6, worin Ra eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe bedeutet.

8. Verbindungen gemäss einem der Ansprüche 3-6, worin Ra die Gruppe -NRR' und R und R' je Wasserstoff oder niederes Alkyl oder zusammen mit dem Stickstoffatom einen gegebenenfalls durch eine oder zwei niedere Alkylgruppen substituierten, 5- oder 6- gliedrigen, gesättigten Heterocyclus, der ein oder zwei Stickstoffatome oder ein Stickstoffatom und ein Sauerstoff- oder Schwefelatom als Heteroatom enthält, bedeuten.

9. Verbindungen gemäss Anspruch 7, worin Ra eine unsubstituierte oder durch niederes Alkyl, niederes Alkoxy oder Halogen mono-substituierte Phenylgruppe bedeutet.

10. Verbindungen gemäss Anspruch 8, worin R und R' je niederes Alkyl oder zusammen mit dem Stickstoffatom 4-Morpholinyl oder 1-Piperidinyl bedeuten.

11. Verbindungen gemäss einem der Ansprüche 1-10, worin $R^5$ Wasserstoff bedeutet.

12. Verbindungen gemäss einem der Ansprüche 1-11, worin $R^4$ die Gruppe $-N=CR^6-Q-NR^1R^2$ bedeutet.

13. Verbindungen gemäss einem der Ansprüche 1-11, worin $R^4$ die Gruppe -CHRc-CO-Re bedeutet.

14. Verbindungen gemäss Anspruch 13, worin Rc Wasserstoff bedeutet.

15. Verbindungen gemäss Anspruch 13 oder 14, worin Re niederes Alkyl, niederes Alkoxy oder eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe bedeutet.

16. Verbindungen gemäss Anspruch 15, worin Re niederes Alkyl bedeutet.

17. Verbindungen gemäss einem der Ansprüche 1-16, worin Q 1,4-Phenylen bedeutet, das noch durch einen oder zwei Substituenten aus der Gruppe bestehend aus niederem Alkyl und niederem Alkoxy substituiert sein kann.

18. Verbindungen gemäss Anspruch 17, worin Q 1,4-Phenylen bedeutet.

19. Verbindungen gemäss einem der Ansprüche 1-18, worin $R^1$ und $R^2$ je niederes Alkyl bedeuten.

20. Verbindungen gemäss einem der Ansprüche 1 bis 19, worin $R^6$ Wasserstoff bedeutet.

21. 1,3-Bis[[p-(Dimethylamino)benzyliden]amino]-2-[2-(dimethylamino)vinyl]imidazoliumchlorid.

22. 1,3-Bis[[p-(Dimethylamino)benzyliden]amino]-2-(2-piperidinovinyl)imidazoliumchlorid.

23. 1,3-Bis[[p-(Dimethylamino)benzyliden]amino]-2-(2-morpholinovinyl)imidazoliumchlorid.

24. 1,3-Bis[[p-(Dimethylamino)benzyliden]amino]-2-phenylimidazoliumchlorid.

25. 1,3-Bis[[p-(Dimethylamino)benzyliden]amino]-2-(p-chlorphenyl)imidazoliumchlorid.

26. 3-[[p-(Dimethylamino)benzyliden]amino]-1-(pivaloylmethyl)-2-(p-tolyl)imidazoliumbromid.

27. 3-[[p-(Dimethylamino)benzyliden]amino]-1-(pivaloylmethyl)-2-(m-methoxyphenyl)imidazoliumbromid.

28. Verbindungen der allgemeinen Formel

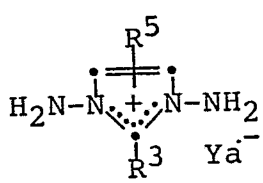

II

worin $R^5$ Wasserstoff, niederes Alkyl, niederes Hydroxyalkyl, niederes Alkoxyalkyl, niederes Halogenalkyl, Aryl oder einen ankondensierten Benzolring, $R^3$ niederes Alkylthio, niederes Alkoxy oder die Gruppe $-(A)_n-Ra$, n die Zahl 0 oder 1, A Vinylen oder niederes Alkylen, Ra Aryl, Heteroaryl oder eine basische Aminogruppe, die gestrichelte Linie eine zusätzliche Doppelbindung und $Ya^-$ ein Anion bedeuten.

29. Verbindungen der allgemeinen Formel

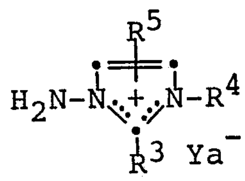 III

worin $R^5$ Wasserstoff, niederes Alkyl, niederes Hydroxyalkyl, niederes Alkoxyalkyl, niederes Halogenalkyl, Aryl oder einen ankondensierten Benzolring, $R^3$ niederes Alkylthio, niederes Alkoxy oder die Gruppe $-(A)_n$-Ra, n die Zahl 0 oder 1, A Vinylen oder niederes Alkylen, Ra Aryl, Heteroaryl oder eine basische Aminogruppe, $R^4$ eine gesättigte oder partiell ungesättigte niedere Kohlenwasserstoffgruppe, eine basische Aminogruppe oder die Gruppe -N=CRc-Rb, -CHRcRd, -NH-CHRcRd, -NH-CO-Re oder -CHRc-CO-Re, Rb Aryl, Heteroaryl oder eine basische Aminogruppe, Rc Wasserstoff oder niederes Alkyl, Rd Aryl oder Heteroaryl, Re Wasserstoff, eine gegebenenfalls über ein Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl- oder basische Aminogruppe, die gestrichelte Linie eine zusätzliche Doppelbindung und $Ya^-$ ein Anion bedeuten.

30. Verbindungen gemäss Anspruch 28 oder 29, worin $R^5$ Wasserstoff bedeutet.

31. Verbindungen der allgemeinen Formel

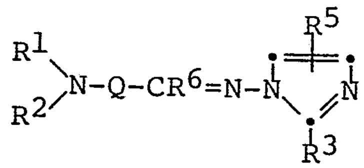

V

worin das Symbol Q Arylen oder Heteroarylen, die Gruppe $-NR^1R^2$ eine basische Aminogruppe, $R^3$ niederes Alkylthio, niederes Alkoxy oder die Gruppe $-(A)_n-Ra$, n die Zahl 0 oder 1, A Vinylen oder niederes Alkylen, Ra Aryl, Heteroaryl oder eine basische Aminogruppe, $R^5$ Wasserstoff, niederes Alkyl, niederes Hydroxyalkyl, niederes Alkoxyalkyl, niederes Halogenalkyl, Aryl oder einen ankondensierten Benzolring und $R^6$ Wasserstoff oder niederes Alkyl bedeuten.

32. Verbindungen gemäss einem der Ansprüche 1-27 zur Anwendung als therapeutische Wirkstoffe.

33. Verbinungen gemäss einem der Ansprüche 1-27 zur Anwendung als antibakteriell, antimykotisch, protozoozid und/oder anthelmintisch wirksame Stoffe.

34. Verbindungen gemäss einem der Ansprüche 1-27 zur Anwendung als filarizid wirksame Stoffe.

35. Arzneimittel, enthaltend eine Verbindung gemäss einem der Ansprüche 1-27 und ein therapeutisch inertes Trägermaterial.

36. Antibakteriell, antimykotisch, protozoozid und/oder anthelmintisch wirksame Mittel, enthaltend eine Verbindung gemäss einem der Ansprüche 1-27 und ein therapeutisch inertes Trägermaterial.

37. Verwendung von Verbindungen gemäss einem der Ansprüche 1-27 bei der Bekämpfung oder Verhütung von Krankheiten.

38. Verwendung von Verbindungen gemäss einem der Ansprüche 1-27 bei der Bekämpfung oder Verhütung von Infektionen durch Bakterien, Pilzen, parasitären Protozooen und/oder parasitären Würmern.

39. Verwendung von Verbindungen gemäss einem der Ansprüche 1-27 zur Herstellung von antibakteriell, antimykotisch, protozoozid und/oder anthelmintisch wirksamen Mitteln.

40. Verwendung von Verbindungen gemäss einem der Ansprüche 28 bis 31 zur Herstellung von therapeutischen Wirkstoffen, welche als gemeinsames Strukturmerkmal eine Gruppe der allgemeinen Formel

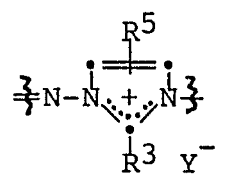

$Y^-$

worin $R^3$, $R^5$, $Y^-$ und die gestrichelte Linie die in Anspruch 1 angegebene Bedeutung besitzen,
aufweisen.

41. Verwendung von Verbindungen gemäss einem der Ansprüche 28 bis 31 zur Herstellung von Verbindungen gemäss einem der Ansprüche 1 bis 27.

42. Verfahren zur Herstellung von Verbindungen der allgemeinen Formel

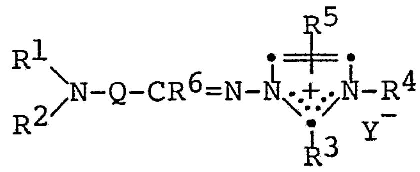 I

worin das Symbol Q Arylen oder Heteroarylen, die Gruppe $-NR^1R^2$ eine basische Aminogruppe, $R^3$ niederes Alkylthio, niederes Alkoxy oder die Gruppe $-(A)_n-Ra$, $R^4$ eine gesättigte oder partiell ungesättigte niedere Kohlenwasserstoffgruppe, eine basische Aminogruppe oder die Gruppe -N=CRc-Rb, -CHRcRd, -NH-CHRcRd, -NH-CO-Re oder -CHRc-CO-Re, $R^5$ Wasserstoff, niederes Alkyl, niederes Hydroxyalkyl, niederes Alkoxyalkyl, niederes Halogenalkyl, Aryl oder einen ankondensierten Benzolring, $R^6$ Wasserstoff oder niederes Alkyl, Ra und Rb je Aryl, Heteroaryl oder eine basische Aminogruppe, Rc Wasserstoff oder niederes Alkyl, Rd Aryl oder Heteroaryl, Re Wasserstoff, eine gegebenenfalls über ein Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl- oder basische Aminogruppe, A Vinylen oder niederes Alkylen, n die Zahl 0 oder 1, die gestrichelte Linie eine zusätzliche Doppelbindung und das Symbol $Y^-$ ein pharmazeutisch annehmbares Anion bedeuten,

und von pharmazeutisch annehmbaren Säureadditionssalzen davon, dadurch gekennzeichnet, dass man

a) eine Verbindung der allgemeinen Formel

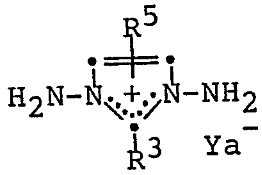 II oder 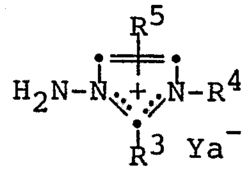 III

worin $R^3$, $R^4$, $R^5$ und die gestrichelte Linie obige Bedeutung besitzen, und $Ya^-$ ein Anion bedeutet,

mit einer Carbonylverbindung der allgemeinen Formel

$$R^1R^2N\text{-}Q\text{-}CO\text{-}R^6 \qquad IV$$

worin $R^1$, $R^2$, $R^6$ und Q obige Bedeutung besitzen,

umsetzt, oder

b) eine Verbindung der allgemeinen Formel

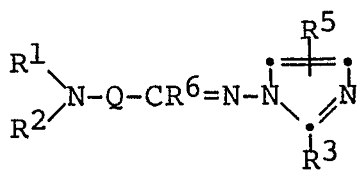 V

worin $R^1$, $R^2$, $R^3$, $R^5$, $R^6$ und Q obige Bedeutung besitzen,

mit einer Verbindung der allgemeinen Formel

$$R^{41}\text{-}X \qquad VI$$

worin $R^{41}$ eine gesättigte oder partiell ungesättigte niedere Kohlenwasserstoffgruppe oder die Gruppe -CHRcRd oder -CHRc-CO-Re und X eine Abgangsgruppe bedeuten, und Rc, Rd und Re obige Bedeutung besitzen,

umsetzt, oder

0253084

c) eine Verbindung der allgemeinen Formel

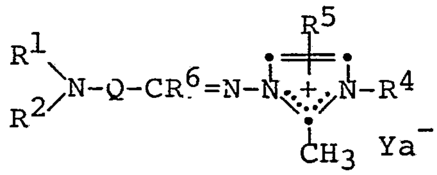

VII

worin $R^1$, $R^2$, $R^4$, $R^5$, $R^6$, Q, und die gestrichelte Linie und $Ya^-$ obige Bedeutung besitzen,
mit einem Aldehyd der allgemeinen Formel

Ra-CH=O VIII

worin Ra obige Bedeutung besitzt,
kondensiert, oder

d) eine Verbindung der allgemeinen Formel

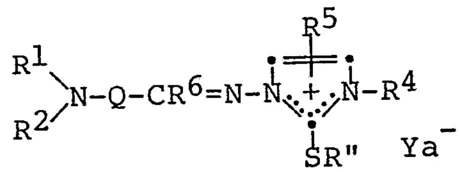

Ib

worin $R^1$, $R^2$, $R^4$, $R^5$, $R^6$, Q, die gestrichelte Linie und $Ya^-$ obige Bedeutung besitzen, und R" niederes Alkyl bedeutet,
mit Ammoniak oder einem primären oder sekundären basischen Amin umsetzt,

e) gegebenenfalls in einer erhaltenen Verbindung das mit $Ya^-$ bezeichnete Anion gegen ein pharmazeutisch annehmbares Anion austauscht und

f) erwünschtenfalls eine erhaltene Verbindung der Formel I in ein pharmazeutisch annehmbares Salz überführt.

43. Verfahren gemäss Anspruch 42, worin $R^3$ niederes Alkylthio bedeutet.

44. Verfahren gemäss Anspruch 42, worin $R^3$ die Gruppe $-(A)_n-Ra$ bedeutet.

45. Verfahren gemäss Anspruch 44, worin n die Zahl 0 bedeutet.

46. Verfahren gemäss Anspruch 44, worin n die Zahl 1 bedeutet.

47. Verfahren gemäss Anspruch 46, worin A Vinylen bedeutet.

48. Verfahren gemäss einem der Ansprüche 44-47, worin Ra eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe bedeutet.

49. Verfahren gemäss einem der Ansprüche 44-47, worin Ra die Gruppe -NRR' und R und R' je Wasserstoff oder niederes Alkyl oder zusammen mit dem Stickstoffatom einen gegebenenfalls durch eine oder zwei niedere Alkylgruppen substituierten, 5- oder 6- gliedrigen, gesättigten Heterocyclus, der ein oder zwei Stickstoffatome oder ein Stickstoffatom und ein Sauerstoff- oder Schwefelatom als Heteroatom enthält, bedeuten.

50. Verfahren gemäss Anspruch 48, worin Ra eine unsubstituierte oder durch niederes Alkyl, niederes Alkoxy oder Halogen mono-substituierte Phenylgruppe bedeutet.

51. Verfahren gemäss Anspruch 49, worin R und R' je niederes Alkyl oder zusammen mit dem Stickstoffatom 4-Morpholinyl oder 1-Piperidinyl bedeuten.

52. Verfahren gemäss einem der Ansprüche 42-51, worin $R^5$ Wasserstoff bedeutet.

53. Verfahren gemäss einem der Ansprüche 42-52, worin $R^4$ die Gruppe $-N=CR^6-Q-NR^1R^2$ bedeutet.

54. Verfahren gemäss einem der Ansprüche 42-52, worin $R^4$ die Gruppe -CHRc-CO-Re bedeutet.

55. Verfahren gemäss Anspruch 54, worin Rc Wasserstoff bedeutet.

56. Verfahren gemäss Anspruch 54 oder 55, worin Re niederes Alkyl, niederes Alkoxy oder eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe bedeutet.

57. Verfahren gemäss Anspruch 56, worin Re niederes Alkyl bedeutet.

58. Verfahren gemäss einem der Ansprüche 42-57, worin Q 1,4-Phenylen bedeutet, das noch durch einen oder zwei Substituenten aus der Gruppe bestehend aus niederem Alkyl und niederem Alkoxy substituiert sein kann.

59. Verfahren gemäss Anspruch 58, worin Q 1,4-Phenylen bedeutet.

60. Verfahren gemäss einem der Ansprüche 42-59, worin $R^1$ und $R^2$ je niederes Alkyl bedeuten.

61. Verfahren gemäss einem der Ansprüche 42 bis 60, worin $R^6$ Wasserstoff bedeutet.

62. Verfahren gemäss Anspruch 42, dadurch gekennzeichnet, dass man 1,3-Bis[[p-(Dimethylamino)benzyliden]-amino]-2-[2-(dimethylamino)vinyl]imidazoliumchlorid herstellt.

63. Verfahren gemäss Anspruch 42, dadurch gekennzeichnet, dass man 1,3-Bis[[p-(Dimethylamino)benzyliden]-amino]-2-(2-piperidinovinyl)imidazoliumchlorid herstellt.

64. Verfahren gemäss Anspruch 42, dadurch gekennzeichnet, dass man 1,3-Bis[[p-(Dimethylamino)benzyliden]-amino]-2-(2-morpholinovinyl)imidazoliumchlorid herstellt.

65. Verfahren gemäss Anspruch 42, dadurch gekennzeichnet, dass man 1,3-Bis[[p-(Dimethylamino)benzyliden]-amino]-2-phenylimidazoliumchlorid herstellt.

66. Verfahren gemäss Anspruch 42, dadurch gekennzeichnet, dass man 1,3-Bis[[p-(Dimethylamino)benzyliden]-amino]-2-(p-chlorphenyl)imidazoliumchlorid herstellt.

67. Verfahren gemäss Anspruch 42, dadurch gekennzeichnet, dass man 3-[[p-(Dimethylamino)benzyliden]-amino]-1-(pivaloyl-methyl)-2-(p-tolyl)imidazoliumbromid herstellt.

68. Verfahren gemäss Anspruch 42, dadurch gekennzeichnet, dass man 3-[[p-(Dimethylamino)benzyliden]-amino]-1-(pivaloyl-methyl)-2-(m-methoxyphenyl)-imidazoliumbromid herstellt.

***